# EUROPÄISCHE PATENTSCHRIFT

(11) **EP 2 349 567 B1**
(45) Veröffentlichungstag und Bekanntmachung des Hinweises auf die Patenterteilung: **17.09.2014**
(21) Anmeldenummer: 09748747.4
(22) Anmeldetag: 27.10.2009
(51) Int. Cl.: B01L 3/00, C12Q 1/24, G01N 33/569, G01N 35/00

(54) **VORRICHTUNG UND VERFAHREN ZUR ANALYSE VON ZELLEN**
DEVICE AND METHOD FOR ANALYSING CELLS
DISPOSITIF ET PROCÉDÉ D'ANALYSE DE CELLULES

(30) Priorität: 27.10.2008 DE 102008053270
(43) Veröffentlichungstag der Anmeldung: 03.08.2011
(73) Patentinhaber: Medizinische Hochschule Hannover, 30625 Hannover (DE)
(72) Erfinder: HENNIG, Christian, 30659 Hannover (DE); HANSEN, Gesine, 30625 Hannover (DE)
(74) Vertreter: Taruttis, Stefan Georg
(86) Internationale Anmeldenummer: PCT/EP2009/064163
(87) Internationale Veröffentlichungsnummer: WO 2010/049430

(56) Entgegenhaltungen:
- WO-A2-2007/062160
- WO-A2-2007/106598
- US-A1- 2005 250 117
- US-B1- 6 225 109
- US-B1- 7 115 423
- HENNIG C ET AL: "A versatile platform for comprehensiwe Chip-based expioratiwe cytometry" CYTOMETRY PART A 2009 WILEY-LISS INC. USA, Bd. 75, Nr. 4, 2009, Seiten 362-370, XP002562648
- TÁRNOK ATTILA: "Slide-based cytometry for cytomics--a minireview." CYTOMETRY. PART A : THE JOURNAL OF THE INTERNATIONAL SOCIETY FOR ANALYTICAL CYTOLOGY JUL 2006, Bd. 69, Nr. 7, Juli 2006 (2006-07), Seiten 555-562, XP002562649 ISSN: 1552-4922

## Beschreibung

Die Erfindung betrifft eine Vorrichtung und deren Verwendung in einem Verfahren zur Analyse immobilisierter Zellen, insbesondere lebender prokaryotischer und eukaryotischer Zellen, besonders bevorzugt tierischer und menschlicher Zellen. Erfindungsgemäß wird eine Vorrichtung mit einem zumindest teilweise optisch transparenten Kanal bereitgestellt, der im Strahlengang einer optischen Detektionseinrichtung, z.B. eines optischen Mikroskops und/oder einer optischen Abtasteinrichtung mit Laserstrahl (Laserscanner) anzuordnen ist, wobei eine zumindest abschnittsweise Oberflächenbeschichtung des Kanals die effektive, zelltypunspezifische, bzw. nicht zellselektive Adsorption bzw. Immobilisierung lebender Zellen, z.B. aus Kultur, medizinischen Proben oder Biopsien ermöglicht. Die effektive Immobilisierung von Zellen unabhängig vom Zelltyp auf zumindest einem Abschnitt der Oberfläche des Kanals erlaubt die Kontaktierung der immobilisierten Zellen mit Farbstoff - gekoppelten Sonden, auch als Nachweiskonjugat bezeichnet, insbesondere Farbstoff - , gekoppelten Antikörpern, die optische Detektion des Farbstoffs, Inaktivierung des Farbstoffs, und die wiederholte Kontaktierung der immobilisierten Zellen mit einem weiteren Nachweiskonjugat zur aufeinander folgenden Detektion verschiedener Analyten, insbesondere von oberflächengebundenen und/oder zellinternen Antigenen durch den Antigen-spezifischen Bindeanteil des Nachweiskonjugats.

### Stand der Technik

Mahnke und Roederer, (Clin Lab Med. 2007, 27(3) (2007)) beschreiben, wie für die Durchflusszytometrie eine Mehrzahl von Antikörperkonjugaten für den Nachweis von Antigenen auf Zellen aufeinander abgestimmt werden können, um bei deren gleichzeitigen Einsatz Interferenzen der Fluorochromanteile zu vermeiden, die jeweils unterschiedliche Emissionsspektren haben. Zur Trennung überlappender Emissionsspektren, insbesondere bei unspezifischer Anregung eines Fluorochroms durch Energietransfer von einem anderen Fluorochrom, wird eine rechnergestützte Auswertung der Signale vorgeschlagen, bei der unspezifische Emissionssignale als Hintergrund abgezogen werden können.

Laffers et al. (Cytometry Part A 69A: 127-130 (2006)) beschreiben die Analyse von lebenden Zellen per FACS oder Mikroskopie von lysierten, denaturierten Zellen, die nach Inkubation in Suspension mit einem markierten Antikörper auf einen Objektträger aufgetragen und durch Trocknen immobilisiert wurden. Die Antikörper-Fluorochrom-Konjugate wiesen jeweils unterschiedliche Fluorochrome auf. Für die Auswertung wurden die Daten aus der Durchflusszytometrie (FACS) oder Mikroskopie mit Fluoreszenzdetektion computergestützt überlagert.

Tárnok (Cytometry Part A 69A: 555-562 (2006)) erwähnt neben der Zytometrie unter Verwendung von Antikörperkonjugaten mit verschiedenen Fluorochromen die nacheinander folgende Anwendung von Antikörper-Fluorochrom-Konjugaten, das Ausbleichen von Fluorochromen mittels Bestrahlung, die Aktivierung und die Zerstörung von Fluorochromen mittels Strahlung.

Hennig, Adams und Hansen beschreiben in Cytrometry Part A 362-370 (2009) ein Verfahren zur unspezifischen Immobilisierung von Zellen in einer Mikrofluidikkammer und die anschließende Analyse der immobilisierten Zellen mittels Farbstoff - markierter Antikörper.

Die WO 2007/106598 A2 beschreibt ein Verfahren zur Immobilisierung von Zellen in einer Durchflusskammer mit Hilfe von Bindemolekülen, die u.a. Antikörper, Peptide, Nukleinsäuren, Rezeptoren, Liganden, Aptamere, MHC - Moleküle, Biotin, Avidin, Oligonukleotide, Koordinierungskomplexe, synthetische Polymeren oder Kohlenhydrate sein können.

Die US 7,115,423 B1 beschreibt die Anordnung einer Vielzahl von Nukleinsäuresonden auf einem Träger zur spezifischen Hybridisierung mit einem nukleinsäurehaltigen Analyten.

Die US 6,225,109 B1 beschreibt ein Verfahren zum Nachweis von SNPs mittels einer Vielzahl in Reihen immobilisierter Oligonukleotide.

Die WO 2007/062160 A2 beschreibt eine Vorrichtung zur Sequenzierung mit einem Glassträger, auf dem poly-dT-Oligonukleotide zur Hybridisierung von DNA gebunden sind und auf den ein Mikroskop gerichtet ist. Die an die poly-dT-Oligonukleotide hybridisierte DNA wird sequenziert.

Die US 2005/250117 A1 beschreibt einen Mikrofluidikchip, auf dem oligo-dT oder oligo-dA Nukleinsäuren gebunden sind und dort chemisch modifiziert und hybridisiert werden.

Perfetto et al. (Nature 648-654 (2004)) beschreiben Durchflusszytometer für die Detektion einer Vielzahl von Fluorochromen durch jeweils sehr kurz nacheinander erfolgende spezifische Anregung und Wellenlängen-spezifische Detektion emittierten Lichts, was als gleichzeitige Messung bezeichnet wird. Es wird gezeigt, dass verschiedene Fluorochrome, die gleichzeitig in einer Probe vorliegen, bei Anregung mit einer Wellenlänge, die Fluorochrom-spezifisch ist, dennoch unspezifische Emissionen zeigen, was auf einen Energietransfer unter den Fluorochromen zurückgeführt wird. Zur Vermeidung unspezifischer Signale wird die Abstimmung der gleichzeitig einzusetzenden Antikörper-Fluorochrom-Konjugate in einem empirischen Verfahren vorgeschlagen. Zur weiteren Verminderung unspezifischer Emissionssignale wird ein mathematische Kompensation vorgeschlagen, mit der Emissionen herausgerechnet werden, die auf den unspezifischen Energietransfer zwischen Fluorochromen zurückgehen können.

Die US 6,150,173 beschreibt einen rechnergesteuerten Pipettierautomaten, mit dem jeweils nacheinander Antikörper-Fluorochrom-Konjugate mit einer Probe inkubiert werden, nach Einstrahlung einer Anregungswellenlänge die emittierte Strahlung gemessen wird, anschließend das Fluorochrom durch Bestrahlung mit UV zerstört wird, und ein neues Antikörper-Fluorochrom-Konjugat zu der selben Probe gegeben wird. Als Probe werden durch Trocknen, Fixieren mit Aceton und neuerliches Trocknen auf einem Objektträger immobilisierte Zellen verwendet.

Die DE 197 09 348 und die WO 2007/101706 A1 beschreiben, dass vor der Detektion eines spezifischen Antikörper- Fluorochrom-Konjugats die biologische Probe durch Bestrahlung behandelt wird, um unerwünschte Fluoreszenz zu verringern.

Die WO 2007/024701 beschreibt eine Durchflusskammer zur mikroskopischen Beobachtung, innerhalb der halbschalenförmige Erhebungen einen Teil der Querschnittsfläche überdecken und als Reusen zur Aufnahme von Mikropartikeln oder Zellen dienen. Die Agglomeration von Nanopartikeln hält diese an den Reusen fest, sodass die Agglomeration als optischer Indikator verwendet wird.

US 2008/0138848 A1 beschreibt die besondere Gestalt einer Durchflusszelle, in der beispielsweise Hefezellen stationär in der Strömung gehalten werden und mikroskopisch analysiert werden können.

Die WO 2007/106598 beschreibt die Beschichtung von Oberflächen mit spezifischen Bindemolekülen, beispielsweise Antikörpern, um eine Wechselwirkung mit einem Analyten herzustellen, und erwähnt neben Antikörpern auch Nukleinsäuren als spezifisches Bindemolekül für Analyten, ohne die Eignung von Nukleinsäuren zur spezifischen Immobilisierung experimentell zu zeigen. Die Spezifität des Analyseverfahrens beruht dabei auf der einmaligen spezifischen Bindung eines Analyten aus der Probe, so dass zur Detektion nur das Vorliegen der Bindung des Analyten an die Oberfläche festgestellt wird; eine weitere spezifische Detektion von Bestandteilen gebundener Analyten erfolgt nicht. Denn die einzelnen Zellarten werden durch die zellartspezifische Bindung, die durch die Nukleinsäuren erfolgt, an die Oberfläche gebunden werden. Die typspezifisch immobilisierten Zellen stellen daher bereits eine aus der eingesetzten Zellmischung selektierte Untergruppe dar, so dass es für die Analyse ausreicht, die Anwesenheit der hochspezifisch selektierten immobilisierten Zellen nachzuweisen. Überdies wäre eine weitergehende Analyse auf Grund der spezifischen Selektion auf die immobilisierte Untergruppe beschränkt.

Die US 2002/0128234 A1 verwendet derivatisiertes PEG zur Anbindung des Bindemoleküls.

Die bekannten Vorrichtungen zur Immobilisierung von Zellen sind dahingehend nachteilig, dass entweder nur denaturierte Zellen auf einem Trägersubstrat fixiert werden können, oder nur vorbestimmte Zellen spezifisch immobilisiert werden, beispielsweise durch Antikörper, die auf dem Trägersubstrat gebunden sind. Die praxisrelevanten Verfahren zur Analyse von Zellen verwenden jeweils die durchflusszytometrische Detektion spezifisch markierter Zellen, so dass notwendigerweise alle spezifischen Markierungsreagenzien gleichzeitig optisch aktiv und mit den Zellen in Kontakt sein müssen, woraus sich störende Wechselwirkungen der optisch detektierbaren Anteile ergeben.

### Aufgabe der Erfindung

Gegenüber dem vorbekannten Stand der Technik ist es die Aufgabe der vorliegenden Erfindung, eine Vorrichtung und ein Verfahren zur Analyse von Zellen bereitzustellen, wobei die Zellen, insbesondere bakterielle und tierische Zellen, auf einfache Weise lebend auf einem Trägersubstrat immobilisiert werden können.

### Allgemeine Beschreibung der Erfindung

Die Erfindung löst die Aufgabe mit den Merkmalen der Ansprüche, insbesondere durch Verwendung eines Trägersubstrats für die unspezifische Immobilisierung lebender bakterieller und/oder eukaryotischer Zellen, insbesondere tierischer Zellen, die als Einzelzellen, Zellagglomerate oder Gewebsschnitte vorliegen können, insbesondere die Verwendung des Trägersubstrats in einem Analyseverfahren. In einer ersten Ausführungsform ist die Oberfläche des Trägersubstrats zumindest abschnittsweise zwischen den Abstandshaltern mit einer Oligonukleinsäuren aufweisenden oder aus Oligonukleinsäuren bestehenden Schicht versehen, vorzugsweise mit kovalent und/oder mittels Kopplungsgruppen mit einem ihrer Enden (3' oder 5') an das Trägermaterial gekoppelten Ribonukleinsäuren, beispielsweise RNA, vorzugsweise ein- oder zweisträngige DNA. Für die Zwecke der Erfindung umfasst der Begriff der Oligonukleinsäuren natürliche und synthetische Nukleinsäureketten, jeweils einsträngig oder zweisträngig, vorzugsweise mit einer Länge von mindestens 5 nt (Nukleotiden), bevorzugter 10 bis 10.000 nt, noch bevorzugter bis 5000 nt oder bis 1000 nt, besonders bevorzugt 10 bis 100 oder bis 50 nt. Bevorzugt besteht die zumindest abschnittsweise Beschichtung des Trägersubstrats aus chemisch gebundenen ein- und/oder zweisträngigen Oligonukleinsäuren, insbesondere DNA. Die Oligonukleinsäuren haben erfindungsgemäß eine Nukleotidsequenz, die in Lösung, z.B. unter zellphysiologischen Bedingungen im Wesentlichen eine lineare Konformation einnimmt. Geeignete Oligonukleinsäuren weisen daher auf oder bestehen aus einer Abfolge von zumindest 90%, bevorzugt 99% Nukleotiden, die jeweils dasselbe von A (Adenin), T (Thymin), G (Guanin) C (Cytosin), I (Inosin) oder U (Uridin) aufweisen oder aus deren Nukleotiden bestehen, z.B. oligo-A, oligo-dA, oligo-T, oligo-dT, oligo-C, oligo-dC, oligo-G, oligo-dG, oligo-I, oligo-dI, oligo-U, oligo-dU mit weniger als 10%, vorzugsweise weniger 1% der Gesamtnukleotidzahl andere Nukleotide, insbesondere bevorzugt aus einer Abfolge von Nukleotiden, die jeweils die selbe Base aufweisen, z.B. aus jeweils Nukleotiden, die A oder T enthalten, besonders bevorzugt nur aus einer Abfolge von einem der Nukleotide A oder dA bzw. aus T oder dT bestehen, und z.B. ein Homooligomer bilden. Die vorgenannten Oligonukleinsäuren sind vorzugsweise einzelsträngig.

Für den Fall, dass die Oligonukleinsäuren eine doppelsträngige Konformation haben, weisen sie auf oder bestehen aus aneinandergrenzenden Abschnitten, von denen ein erster Abschnitt aus oligo-A und/oder oligo-dA besteht, und ein zweiter Abschnitt aus oligo-T und/oder oligo-dT. Erster und zweiter Abschnitt weisen jeweils vorzugsweise weniger als 10%, vorzugsweise weniger 1% andere Nukleotide an der Gesamtzahl von Nukleotiden auf, und bestehen vorzugsweise aus einer Abfolge nur jeweils desselben Nukleotids. Bevorzugt bestehen Abschnitte aus 3 bis 100, vorzugsweise 5 bis 50 Nukleotiden. Zwischen erstem und zweitem Abschnitt sind maximal 10, bevorzugt maximal 2 bis 5, besonders bevorzugt kein anderes Nukleotid angeordnet. Die ersten und zweiten Abschnitte können auf einem gemeinsamen Nukleotidstrang angeordnet sein, oder auf 2 komplementären hybridisierten Strängen. Vorzugsweise weisen die ersten und zweiten Abschnitte dieselbe Anzahl Nukleotide auf, besonders bevorzugt besteht ein doppelsträngiges Oligonukleotid aus einer Abfolge von ersten und komplementären zweiten Abschnitten, die jeweils dieselbe Nukleotidanzahl aufweisen und symmetrisch zu dem oder den mittigen Nukleotiden des Oligonukleotids angeordnet sind, insbesondere aus einem ersten Abschnitt und einem komplementären zweiten Abschnitt.

Es hat sich gezeigt, dass Oligonukleinsäuren dann besonders zur Verwendung für die Immobilisierung lebender tierischer Zellen unter physiologischen Bedingungen geeignet sind, wenn die Oligonukleinsäuren eine lineare oder fadenförmig gestreckte Konformation haben, also entsprechend im Wesentlichen z.B. keine dreidimensionale Faltung aufweisen, wie sie z.B. bei Aptameren auftritt. Die Oligonukleinsäuren mit linearer Konformation sind vorzugsweise mit einem ihrer Enden am Trägersubstrat gebunden, während das andere Ende ungebunden ist. Es wird gegenwärtig angenommen, dass die Oligonukleinsäuren wegen der Kombination ihrer linearen Konformation mit der Ausbildung einer Bindung nur eines ihrer Enden zum Trägersubstrat in wässrigem Milieu etwa senkrecht und fadenförmig zum Trägersubstrat angeordnet sind. Die Bindung des einen Endes der Oligonukleinsäuren am Trägersubstrat ist vorzugsweise kovalent, z.B. über zumindest divalent kovalent bindende Kopplungsgruppen, alternativ mittels Kopplungsgruppen, von denen z.B. ein erster Bindungspartner mit dem Trägersubstrat verbunden ist, und ein spezifisch an den ersten bindender zweiter Bindungspartner an eine Oligonukleinsäure gebunden ist. Eine bevorzugte Kombination erster und zweiter Bindungspartner sind Avidin oder Streptavidin mit Biotin. Als Kopplungsgruppe kann auch ein Nukleotid endständig an der Oligonukleinsäure angeordnet sein, welches z.B. kovalent mittels Zwischengruppen oder mit zusätzlichem erstem und zweitem Bindungspartner mit dem Trägersubstrat verbunden ist. Ein an der Oligonukleinsäure angeordnetes Nukleotid, das mit dem Trägersubstrat verbunden ist, kann optional eine zweite erfindungsgemäße Oligonukleinsäure tragen, so dass z.B. abhängig von der Kopplungsgruppe auch zwei oder mehrere Oligonukleinsäuren an eine Kopplungsgruppe gebunden sein können.

Es hat sich gezeigt, dass die Zellen unspezifisch in dem Abschnitt des Trägersubstrats immobilisiert sind, in dem erfindungsgemäß Oligonukleinsäuren linearer Konformation mit einem ihrer Enden gebunden sind.

Vorzugsweise ist das Trägersubstrat silikathaltig, beispielsweise silikathaltiges Glas, besonders bevorzugt Borosilikatglas.

Bevorzugt sind die Oligonukleinsäuren in der Erfindung mit einer Anzahl von zumindest 5000/µm², bevorzugt zumindest 7000/µm², noch bevorzugter zumindest 10.000/µm² Trägersubstrat gebunden. Denn es hat sich gezeigt, dass die zelltypunspezifische Immobilisierung von Zellen von der Anzahl Oligonukleinsäuren pro Flächeneinheit Trägersubstrat abhängt, und mit zunehmender Anzahl effektiver ist, bzw. die Zellbindung unterhalb dieser Anzahl / Flächeneinheit für die vom Zelltyp unabhängige Immobilisierung der Erfindung nicht ausreicht bzw. nicht effizient erfolgt.

Optional zusätzlich zu der zumindest abschnittsweisen Beschichtung des Trägersubstrats mit gebundenen Oligonukleinsäuren innerhalb des Kanals weist das Trägersubstrat einen Rahmen auf, der als zur Probe, beispielsweise einem Gewebsschnitt, formschlüssiger Vorsprung auf dem Trägersubstrat angeordnet ist. Durch einen solchen Rahmen auf dem Trägersubstrat können Zellen, die in einem Gewebsschnitt enthalten sind, formschlüssig positionsgenau auf dem Trägersubstrat immobilisiert sein, sodass lebende Zellen innerhalb des Gewebsschnitts wiederholt analysiert werden können, wobei jeweils positionsgenau einzelne Abschnitte des Gewebsschnitts wiederholt optisch analysiert werden können, auch wenn das Trägersubstrat zwischen einzelnen Detektionsschritten aus dem Gesichtsfeld des zur Analyse verwendeten Mikroskops entfernt wird. Denn sowohl die formschlüssige Halterung eines Gewebsschnitts auf dem Trägersubstrat, als auch die Immobilisierung von Zellen auf der mit Oligonukleinsäuren beschichteten Oberfläche des Trägersubstrats erfolgt unabhängig vom Zelltyp, daher nicht zellselektiv, und positionsgenau, sodass die Positionen immobilisierter Zellen in den Ausführungsformen der Erfindung in Relation zum Trägersubstrat positionsgenau und damit wiederholt analysierbar sind. Entsprechend ist das Analyseverfahren mit dem als Trägersubstrat verwendeten, mit kovalent gebundenen Oligonukleinsäuren beschichtete Silikatglas vorzugsweise zyklisch, wobei in jedem Zyklus, der zumindest die Schritte des Kontaktierens mit Antikörperkonjugat, des Detektierens von dessen Fluorochromanteil und des Unwirksammachens des Fluorochromanteils enthält oder daraus besteht, ein Nachweiskonjugat mit anderer Antigenspezifität, insbesondere ein Antikörperkonjugat verwendet wird und die Zellen lebend immobilisiert oder denaturiert bzw. perforiert fixiert sind.

Anstelle eines Antikörpers wie Antikörperkonjugate können Nachweiskonjugate eine andere Bindegruppe als einen Antikörper aufweisen, die an ein Fluorochrom gekoppelt ist. Entsprechend umfasst der Begriff der Antikörperkonjugate für die Zwecke der Erfindung auch Fluorochrom-gekoppelte Bindemoleküle, die für einen Bestandteil einer Zelle spezifisch sind. Solche Bindemoleküle können ausgewählt sein aus synthetischen ein- oder mehrkettigen Antikörpern, Aptameren (Oligonukleinsäuren, die spezifisch an ein Epitop binden), Lektinen und rezeptorspezifischen Bindemolekülen.

Dabei hat die Beschichtung von Silikatglas mit Oligonukleinsäuren mit im wässrigen Milieu linearer Konformation den Vorteil, eine hohe Dichte und gleichmäßige Verteilung immobilisierter lebender Zellen bei Kontaktierung mit suspendierten Zellen zu erzeugen. Zusätzliche Reagenzien sind für die Immobilisierung prokaryotischer und/oder eukaryotischer Zellen auf der mit kovalent gebundenen Oligonukleinsäuren beschichteten Silikatglasoberfläche nicht erforderlich, so dass das Verfahren zur Immobilisierung aus der Kontaktierung der mit Oligonukleinsäuren beschichteten Silikatglasoberfläche mit Zellen oder Zellagglomeraten oder Gewebsstücken in Suspension bestehen kann, wobei die Suspension wässrig mit einem für die Zellen verträglichen Salzgehalt sein kann, insbesondere ein zellphysiologisches Medium, wahlweise ohne organische Bestandteile.

Ein besonderer Vorteil der erfindungsgemäßen Immobilisierung lebender eukaryotischer, insbesondere tierischer und menschlicher Zellen auf einem Trägersubstrat liegt gegenüber bekannten Immobilisierungsverfahren darin, dass bei der erfindungsgemäßen Oligonukleotidbeschichtung eine nicht zelltypspezifische Immobilisierung erfolgt, sondern vielmehr eine unspezifische Immobilisierung von prokaryotischen und eukaryotischen Zellen, z.B. von Bakterien und insbesondere von tierischen einschließlich menschlicher Zellen. Die erfindungsgemäße Beschichtung erlaubt die Immobilisierung von tierischen Zellen über einen langen Zeitraum, beispielsweise mindestens 4h, vorzugsweise mindestens 8 bis 10 h, wobei die Zellen in Zellkulturmedium innerhalb des Kanals lebensfähig sind, der vom Trägersubstrat, dem beabstandeten Deckel und zwei Abstandshaltern gebildet wird. Die Abstandshalter erstrecken sich über den Abstand zwischen Trägersubstrat und Deckel, wobei der zwischen Trägersubstrat, Deckel und Abstandshaltern gebildete Kanal an einem, vorzugsweise an zwei beabstandeten Stellen, vorzugsweise einem ersten und einem gegenüberliegenden zweiten Ende, Öffnungen zum Ein- bzw. Auslass flüssiger Zusammensetzungen aufweist. Der Querschnitt des Kanals kann frei gewählt sein, vorzugsweise sind Trägersubstrat und beabstandet gegenüberliegender Deckel im Überdeckungsbereich parallel zueinander. Die Abstandshalter, die den Abstand zwischen Trägersubstrat und Deckel unter Bildung zumindest einer, vorzugsweise jeweils einer Einlassund einer Auslassöffnung überdecken, können auf dem Trägersubstrat und/oder dem Deckel angebracht oder integral mit einem dieser geformt sein, so dass eine oder zwei Öffnungen, durch die das Innenvolumen des Kanals zugänglich ist, im Deckel und/oder im Trägersubstrat angeordnet sind. Das Trägersubstrat ist vorzugsweise optisch transparent und in Form einer Platte, alternativ in Partikelform. Besonders bevorzugt ist auch der Deckel optisch transparent.

Überdies hat sich gezeigt, dass die Verwendung des erfindungsgemäßen, mit gebundenen Oligonukleinsäuren versehenen Trägersubstrats im Analyseverfahren dazu führt, dass immobilisierte tierische Zellen ihre dreidimensionale elliptische Form oder Kugelform des suspendierten Zustands im Wesentlichen beibehalten, so dass diese immobilisierten Zellen bei Detektion eines Oberflächenantigens in der mikroskopischen Aufnahme ein verstärktes Signal für die parallel zur Betrachtungsrichtung liegenden Zellwandabschnitte zeigen, das eine ringförmige oder ovale Umfangslinie hervorhebt. Im Unterschied dazu zeigen herkömmlich immobilisierte Zellen keine Signalverstärkung durch Überlagerung im Randbereich, sondern vielmehr ein etwa gleichmäßiges Signal über die Fläche, d.h. ohne Signalverstärkung im Randbereich der Aufsicht. Aus diesen Beobachtungen wird gegenwärtig geschlossen, dass die Verwendung des erfindungsgemäßen, mit Oligonukleinsäuren versehenen Trägersubstrats zur Immobilisierung und Analyse von Zellen die sphärische Zellgestalt beibehält, während herkömmliche Trägersubstrate die Zellgestalt abflachen.

Die Kontaktierung der immobilisierten Zellen innerhalb des Kanals ist durch eine Flüssigkeitsströmung entlang des Kanals möglich, beispielsweise durch Aufgeben einer wässrigen Zusammensetzung mit einem spezifischen Nachweiskonjugat, z.B. einem Farbstoff - gekoppelten Antikörper an einer ersten Öffnung des Kanals und Austretenlassen der wässrigen Zusammensetzung aus der gegenüberliegenden zweiten Öffnung des Kanals, vorzugsweise unterstützt durch Entfernen austretender Flüssigkeit. Aufgrund des erfindungsgemäß geringen Innenvolumens des Kanals, beispielsweise im Bereich von 1 bis 200 µL, vorzugsweise 2 bis 20 µL, sind Volumina von 0,5 µL bis 200 µL, vorzugsweise 1 bis 10 µL der wässrigen Zusammensetzungen mit einem Gehalt an Nachweiskonjugat, z.B. Farbstoff - gekoppeltem Antikörper, ausreichend, um den immobilisierten Zellen den ersten und, nach dessen Detektion und Ausbleichen ein weiteres Nachweiskonjugat zuzuführen.

Eine erste Öffnung des Kanals wird an einem ersten Ende des Kanals gebildet, z.B. durch die Querschnittsfläche, die von dem Trägersubstrat und dem beabstandeten Deckel sowie zwei Abstandshaltern aufgespannt wird, während im gegenüberliegenden zweiten Ende des Kanals eine zweite Öffnung gebildet wird, z.B. durch die Querschnittsfläche des Kanals, die zwischen Trägersubstrat und Deckel sowie die zwischen diesen angeordneten Abstandshaltern aufgespannt wird. Vorzugsweise sind Abstandshalter, die den Querschnitt des Kanals begrenzen, mit dem Deckel verbunden, während das Trägersubstrat mit der Beschichtung mit Oligonukleinsäuren gegen die Abstandshalter angeordnet ist, wahlweise nur durch kraftschlüssige Anordnung oder durch formschlüssige Anordnung und/oder mit zwischen Abstandshaltern und Trägersubstrat angebrachtem Klebstoff. Vorzugsweise ist das Trägersubstrat beim Analyseverfahren horizontal und oberhalb des Deckels angeordnet, insbesondere während der Detektion, so dass die mit Oligonukleotiden beschichtete Oberfläche des Trägersubstrats die obere Begrenzung des Kanals bildet und z.B. am Trägersubstrat immobilisierte Zellen in den Kanal zwischen Trägersubstrat und Deckel hängen.

In Ausführungsformen, in denen ein zu einer Gewebsprobe formschlüssiger Rahmen auf dem Trägersubstrat angeordnet ist, hat der Rahmen vorzugsweise eine Höhe von 0,8 bis 5 mal der Schichtdicke der Gewebsprobe, vorzugsweise eine Höhe von 1 bis 1,5 -mal der Schichtdicke der Gewebsprobe, wobei der Deckel in einem Abstand zu Gewebsprobe und Rahmen angeordnet ist. Die Oberflächen des Rahmens sind vorzugsweise senkrecht zu der mit Oligonukleinsäure beschichteten Oberfläche des Trägersubstrats angeordnet. Auf diese Weise ermöglicht der formschlüssig um die Gewebsprobe angeordnete Rahmen den Durchtritt wässriger Zusammensetzungen durch den Kanal, der zwischen Trägersubstrat und Deckel gebildet ist, sodass diese, beispielsweise Waschlösungen und Antikörperkonjugate, die an einer Einlassöffnung in den Kanal einströmen gelassen werden, die Gewebsprobe kontaktieren können, wobei vorzugsweise an einer der Einlassöffnung gegenüberliegenden Auslassöffnung wässrige Zusammensetzung ausströmen gelassen wird, vorzugsweise unterstützt durch Absaugen.

Alternativ zur Immobilisierung lebender Zellen und/oder von Gewebe kann das erfindungsgemäß mit einer Beschichtung von Oligonukleinsäuren und/oder mit einem zur Gewebsprobe formschlüssigen Rahmen versehene Trägersubstrat zur Immobilisierung denaturierter und/oder perforierter Zellen und Gewebsproben verwendet werden. Vorzugsweise werden beim erfindungsgemäßen Verfahren auf dem mit Oligonukleinsäuren und/oder mit einem Rahmen versehenen Trägersubstrat lebende Zellen oder Gewebsschnitte immobilisiert, die nach Analyse, beispielsweise nach einem Zeitraum von 1 bis 10 h analysiert wurden, die optional zusätzlich im immobilisierten Zustand denaturiert und/oder perforiert wurden, beispielsweise durch Zugabe von Formalin und/oder Aceton und/oder Puffer mit Saponin (erhältlich als FixPerm von BD Biosciences) und/oder Methanol.

Alternativ zu lebenden bzw. toten und nicht denaturierten Zellen können beim erfindungsgemäßen Verfahren fixierte und/oder denaturierte Zellen aus einer Suspension durch Kontaktieren der Suspension mit dem Trägersubstrat auf diesem immobilisiert werden.

Das Trägersubstrat mit einer Beschichtung, die aus Oligonukleinsäuren besteht, optional mit einem zu einer Gewebsprobe formschlüssigen Rahmen, kann daher auch in Analyseverfahren mit Denaturierung und Perforation der immobilisierten Zellen verwendet werden, so dass immobilisierte lebende Zellen im Anschluß an die Analyse auf dem Trägersubstrat im lebenden Zustand optional denaturiert und/oder perforiert werden, und in gleicher Position im. Gesichtsfeld der optischen Detektionseinrichtung wie im lebenden Zustand dann im denaturierten und/oder perforierten Zustand analysiert werden.

Die Beschichtung des silikathaltigen Trägersubstrats mit Oligonukleinsäuren ist vorzugsweise von kovalent unmittelbar und/oder mittels Kopplungsgruppen gebundenen Oligonukleinsäuren gebildet, die in dichter Anordnung auf der Oberfläche des Trägersubstrats angeordnet sind, vorzugsweise in einer im wesentlichen kontinuierlichen Anordnung, also unterbrechungsfrei, zumindest in einem Detektionsabschnitt des Trägersubstrats. Der Detektionsabschnitt des Trägersubstrats liegt innerhalb des Kanals und wird zur optischen Analyse vom Gesichtsfeld, d.h. dem optischen Erfassungsbereich der optischen Detektionseinrichtung erfasst. Besonders bevorzugt ist das Trägersubstrat, das mit kovalent gebundenen Oligonukleinsäuren beschichtet ist, die im Detektionsabschnitt eine kontinuierliche Beschichtung bilden, durch Umsetzung des Trägersubstrats zumindest im Detektionsabschnitt durch Kontaktieren mit Alkoxysilangruppen-haltigen Oligonukleotiden in aprotischer Phase, z.B. in organischer Phase erhalten. Denn es hat sich herausgestellt, dass die Kontaktierung einer Silikatoberfläche in organischer Phase mit Alkoxysilangruppen-haltigen Oligonukleotiden die kovalente Bindung von Oligonukleotiden an die Silikatoberfläche ergibt. Vorzugsweise weist die Silikatoberfläche mit Alkoxysilan, insbesondere Methoxysilan, reaktionsfähige Hydroxylgruppen auf, die z.B. durch Anätzen zur Vorbehandlung erhältlich sind, beispielsweise durch Kontaktierung mit wässriger Fluorwasserstoffsäure (HF, Flusssäure).

Vorzugsweise umfasst das Analyseverfahren bzw. die Herstellung von Trägersubstraten für die Verwendung in dem Analyseverfahren die folgenden Schritte:
Erzeugen reaktionsfähiger Hydroxylgruppen auf einem silikathaltigen Trägersubstrat durch Kontaktieren des Trägersubstrats mit einer starken Säure, insbesondere in wässriger Lösung, z.B. ausgewählt aus HF und/oder, optional rauchender, Schwefelsäure,
Austausch der starken Säure gegen ein organisches aprotisches Lösungsmittel, z.B. ausgewählt unter Ether und Aceton,
Entfernen des organischen aprotischen Lösungsmittels und Kontaktieren des Trägersubstrats mit Oligonukleinsäuren, die an einem oder beiden Enden eine Alkoxysilangruppe aufweisen, in alkoholischer Zusammensetzung, z.B. in Methanol,
Waschen mit wässriger Zusammensetzung, z.B. Wasser oder einem zellphysiologischen Medium,
wobei diese Schritte ohne unmittelbare Kontaktierung des Trägersubstrats durch Luftsauerstoff und unmittelbar aufeinander erfolgen, d.h. ohne Trocknen des Trägersubstrats z.B. durch zwischenzeitliches Entfernen eines Flüssigkeitsfilms von dem Trägersubstrat. Auch die Verwendung zur Immobilisierung bzw. zur Zellanalyse, d.h. die Kontaktierung des Trägersubstrats mit Zellen in wässriger Zusammensetzung, erfolgt vorzugsweise ohne unmittelbare Kontaktierung des Trägersubstrats durch Luftsauerstoff und unmittelbar aufeinander, d.h. ohne Trocknen des Trägersubstrats z.B. durch zwischenzeitliches Entfernen eines Flüssigkeitsfilms von dem Trägersubstrat.

Es hat sich gezeigt, dass die Aufrechterhaltung eines Flüssigkeitsfilms während der vorgenannten Schritte zur Herstellung des mit Oligonukleinsäuren versehenen Trägersubstrats und bei der Kontaktierung mit suspendierten Zellen, bzw. in der anschließenden Analyse der auf dem Trägersubstrat immobilisierten Zellen, die Effizienz der Immobilisierung, z.B. die Dichte immobilisierter Zellen, erhöht und z.B. die natürliche Kugelform der Zellen im immobilisierten Zustand und/oder die Genauigkeit der Analyse fördert.

Die mit kovalent gebundenen Oligonukleinsäuren beschichteten Silikatglasoberflächen haben bei der Verwendung zur Immobilisierung von Zellen den Vorteil, dass Zellen durch Kontaktieren ohne weitere reaktive Zusätze in reproduzierbar hoher Dichte im lebenden Zustand immobilisiert werden.

Diese reproduzierbare Dichte der Immobilisierung von Zellen tritt im wesentlichen unabhängig vom Bindungsverhalten des Silikatglases auf, das dieses ohne die Beschichtung mit Oligonukleinsäuren aufweist, so dass durch die Beschichtung im wesentlichen unabhängig von den Bindungseigenschaften des unbeschichteten Silikatglases eine hohe und reproduzierbare Bindungswirkung für Zellen erzeugt wird.

Die Oligonukleinsäuren, insbesondere die mit einer Alkoxysilangruppe verbundenen Oligonukleinsäuren, z.B. Methoxysilangruppen-haltige Oligonukleinsäuren umfassen Nukleinsäuremoleküle oder Desoxyribonukleinsäuremoleküle mit mindestens 5 bis mindestens 50, vorzugsweise mindestens 10 bis mindestens 10000 Nukleotiden, die z.B. synthetisiert sind oder aus biologischem Material isolierte DNA und/oder RNA sind. Die kovalent an die Oligonukleinsäure gebundene Alkoxysilangruppe ist vorzugsweise eine di-oder tri-Alkoxysilangruppe und kann durch herkömmliche Syntheseverfahren mit den Oligonukleinsäuren verbunden werden, z.B. mittels Kopplungsgruppen, die zwischen einer endständigen Phosphatgruppe der Oligonukleinsäure und einer Alkoxysilangruppe angeordnet sind.

Mit kovalent gebundenen Oligonukleinsäuren beschichtetes Silikatglas, z.B. Borosilikatglas, ist durch Kontaktieren eines silikathaltigen Trägersubstrats, das reaktive Hydroxylgruppen aufweist, unter aprotischen Bedingungen mit Oligonukleinsäure erhältlich, die mit Hydroxylgruppen reaktive Gruppen aufweist, z.B. in einem Verfahren, das die Schritte enthält:
Kontaktierung des silikathaltigen Trägermaterials mit Flusssäure, vorzugsweise 0,1 bis 10 Gew.-% in Wasser,
Entfernen der Flusssäure vom Trägersubstrat,
Kontaktieren des Trägersubstrats mit aprotischem, insbesondere organischem Lösungsmittel und
Kontaktieren des Trägersubstrats mit Oligonukleinsäuren, die zumindest eine reaktive Silikatgruppe enthalten, beispielsweise Alkoxysilan-haltige Oligonukleinsäuren, insbesondere Methoxysilan - haltige Oligonukleinsäuren, beispielsweise Methoxysilan-konjugierte Oligonukleinsäuren, in aprotischem, insbesondere organischem Lösungsmittel.
Die Alkoxysilangruppe ist vorzugsweise eine C₁- bis C₆-Alkoxysilangruppe, insbesondere eine Methoxysilangruppe oder Ethoxysilangruppe. Generell weist die Oligonukleinsäure dann z.B. eine Alkoxysilangruppe mit der folgenden Struktur auf:

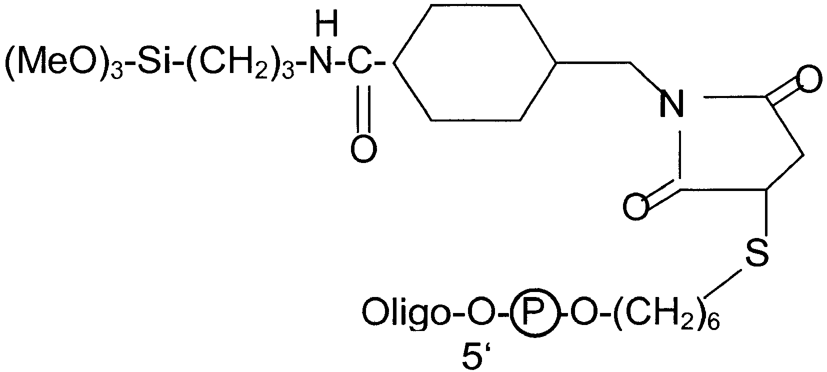

Vorzugsweise wird das mit kovalent gebundenen Oligonukleinsäuren beschichtete silikathaltige Trägersubstrat, z.B. Silikatglas, während des Herstellungsverfahrens, während der Lagerung bis zur Verwendung zur Immobilisierung und während der Immobilisierung und des Analyseverfahrens zumindest in dem mit Oligonukleinsäuren beschichteten Abschnitt in Kontakt mit Flüssigkeit gehalten. Denn es hat sich gezeigt, dass das Trockenen des mit Oligonukleinsäuren beschichteten Abschnitts des Silikatglases zu einer Erhöhung der unspezifischen Bindung von Antikörperkonjugaten führt, die im Analyseverfahren verwendet werden.

Das Entfernen der Flusssäure vom Trägersubstrat und das anschließende Kontaktieren mit organischem Lösungsmittel, das vorzugsweise ein C₁- bis C₆-Alkohol ist, besonders bevorzugt Methanol, Ethanol und/oder Aceton, kann durch Verdrängen der Flusssäure, die vorzugsweise in wässriger Lösung vorliegt, durch das organische Lösungsmittel erfolgen. Die Beschichtung des silikathaltigen Trägersubstrats mit Oligonukleinsäuren bildet eine hochdichte, vorzugsweise monomolekulare Schicht von Oligonukleinsäuren auf dem Trägersubstrat, die eine hohe Kapazität zur Immobilisierung von Zellen, insbesondere tierischer Zellen unabhängig vom Zelltyp und/oder unabhängig von Oberflächenmarkern der Zellen aufweist. Daher ermöglicht die Oligonukleinsäure - Beschichtung des Trägersubstrats die nicht zellspezifische Immobilisierung lebender Zellen, insbesondere bakterieller und tierischer Zellen, sodass bei Analyse einer biologischen Probe jede darin enthaltene Zellart entsprechend ihres Anteils an der Probe immobilisiert wird, und die Immobilisierung keine zellspezifische Selektion ergibt. Dies ist insbesondere für die Analyse von Oberflächenmarkern von Zellen vorteilhaft, da der spezifische Nachweis von Oberflächenmarkern auf Zellen unabhängig von der Immobilisierung der Zellen auf dem Trägersubstrat gewählt werden kann, nämlich allein durch Auswahl einer markerspezifischen Sonde, vorzugsweise Auswahl des Antikörperanteils eines Antikörperkonjugats mit Fluorochromanteil.

Gegenüber bekannten Verfahren zur Immobilisierung von Zellen, die jeweils auf die zelltypspezifische Immobilisierung ausgerichtet sind, hat die mit Oligonukleinsäuren und wahlweise zusätzlich mit einem zur Gewebsprobe formschlüssigen Rahmen versehene Oberfläche des Trägersubstrats eine wesentlich höhere Kapazität zur Bindung lebender Zellen; weiterhin ist die Immobilisierung stabil, beispielsweise über einen Zeitraum von bis zu 8 h für lebende Zellen, sodass eine mehrfache Kontaktierung der immobilisierten Zellen durch wiederholtes Durchströmenlassen von Antikörperkonjugaten in wässriger Zusammensetzung, die jeweils unterschiedliche Antigenspezifitäten haben, im Wesentlichen ohne Zellverlust möglich ist.

Ein Vorteil des mit Oligonukleinsäure beschichteten silikathaltigen Trägersubstrats liegt darin, dass die Immobilisierung ohne aufwendige Probenvorbereitung erfolgen kann, z.B. bei Blut nach Behandlung nur durch Erythrolyse, vorzugsweise nach Abtrennung lysierter Erythrozyten-Bestandteile von den intakten Zellen durch Zentrifugation, oder nach Probenbehandlung durch Isolierung eines gewünschten Zelltyps, z.B. durch Ficoll-Gradientenzentrifugation, Durchflußzytometrie-Verfahren oder Sortierverfahren mit zellspezifischen magnetischen Partikeln (MACS, Miltenyi Biotech). Andere medizinische Proben, die keine Verunreinigungen oder Erythrozyten enthalten, konnten ohne Aufbereitung der Probe unmittelbar immobilisiert werden, z.B. Liquor, Aszites, bronchio-alveoläre Spülung, Feinnadel-Punktat, Gewebe- oder Organbiopsien, optional nach Zellvereinzelung.

Vorzugsweise ist das Trägersubstrat zur Detektion an einer optischen Detektionseinrichtung so angeordnet, dass die mit Oligonukleotiden beschichtete Oberfläche des Trägersubstrats horizontal positioniert ist, vorzugsweise oberhalb des Deckels, sodass immobilisierte Zellen unterhalb des Trägersubstrats angeordnet sind. Zur Detektion ist es bevorzugt, dass sowohl Trägersubstrat als auch Deckel transparent sind.

Das erfindungsgemäße Verfahren zur Detektion von zellgebundenen Antigenen erfolgt durch
- Immobilisierung von Zellen, die Einzelzellen, Zellagglomerate und/oder Gewebsproben sein können, z.B. in Suspension, durch Kontaktierung mit dem mit Oligonukleotiden beschichteten Detektionsabschnitt des Trägersubstrats und/oder durch Anordnung innerhalb eines formschlüssigen Rahmens, der unmittelbar oder beabstandet auf dem Trägersubstrat angeordnet ist, vorzugsweise zur Verwendung mit Gewebsschnitten,
- Kontaktieren der Zellen mit einem ersten Antikörperkonjugat, das eine erste Antigenspezifität und einen Fluorochromanteil aufweist,
- Detektieren von Strahlung, die bei Einstrahlung von Licht mit einer Anregungswellenlänge vom Fluorochromanteil emittiert wird,
- Unwirksammachen bzw. Inaktivieren des Fluorochromanteils, beispielsweise durch Bestrahlung, z.B. mit UV-Licht oder der Anregungswellenlänge des Fluorochroms zum Ausbleichen,
- Kontaktieren der Zellen mit einem zweiten oder weiteren Antikörperkonjugat, das eine zweite bzw. weitere Antigenspezifität und den selben oder einen anderen Fluorochromanteil wie das erste Antikörperkonjugat aufweist, und
- Detektieren der bei Einstrahlung von Licht mit einer Anregungswellenlänge von dem Fluorochromanteil des zweiten Antikörperkonjugats emittierten Strahlung, optional mit Waschen der immobilisierten Zellen nach Kontaktierung mit einem der Antikörperkonjugate, beispielsweise durch Hindurchströmenlassen einer wässrigen Zusammensetzung, beispielsweise von Medium zur Zellkultur und/oder Puffer durch den Kanal, von dem ein Oberflächenabschnitt vom Trägersubstrat gebildet wird,
- Wiederholung der Schritte des Kontaktierens, Detektierens und Unwirksammachens des Fluorochromanteils mit jeweils einem weiteren Antikörperkonjugat.
- Vorzugsweise enthält das Verfahren den Schritt der automatischen Positionsbestimmung von detektierten Fluoreszenzsignalen in Relation zur Position des Trägersubstrats, und
- die virtuelle positionsgenaue Überlagerung von Abbildern detektierter Fluoreszenzsignale, wobei vorzugsweise die Abbilder, die jeweils nach Kontaktierung mit einem Nachweiskonjugat, das insbesondere ein Antikörperkonjugat ist, detektiert wurden, in jeweils einer für das Nachweiskonjugat spezifischen Farbe dargestellt werden

Alternativ zur zyklischen Abfolge des Kontaktierens immobilisierter Zellen mit einem Antikörper-Farbstoff-Konjugat, Entfernen ungebundenen Antikörperkonjugats, Detektion des Antikörperkonjugats und Unwirksammachen des Antikörperkonjugats, kann das Verfahren die Kontaktierung der immobilisierten Zellen mit mindestens 2 oder mehr Antikörperkonjugaten und deren gleichzeitige Detektion umfassen. Bei Verwendung von 2 oder mehr Antikörperkonjugaten haben diese vorzugsweise verschiedene Fluorochromanteile und die Detektion erfolgt bei zumindest zwei Wellenlängen, um verschiedene Fluorochromanteile getrennt voneinander zu detektieren.

Die Verfahrensschritte des Kontaktierens immobilisierter Zellen oder Gewebsstücke mit Antikörperkonjugat, Bestrahlen bei Anregungswellenlänge, Detektion emittierter Strahlung, Unwirksammachen des Fluorochromanteils des Antikörperkonjugats, z.B. durch Ausbleichen, können mehrfach nacheinander mit jeweils unterschiedlichen Antikörperkonjugaten erfolgen, die sich in ihrem Antikörperanteil bzw. ihrer Antigenspezifität unterscheiden, wobei optional der Fluorochromanteil des Antikörperkonjugats jeweils gleich ist. Da beim erfindungsgemäßen Verfahren die Zellen ortsspezifisch am Trägersubstrat immobilisiert sind, wird vorzugsweise das bei der Detektion emittierter Strahlung aufgenommene Abbild mit ortsspezifischer Zuordnung des Abbilds zum Trägersubstrat gespeichert. Die ortsspezifische Zuordnung des Abbilds kann dadurch erfolgen, dass das Trägersubstrat bei der Detektion immer die selbe Position zur optischen Detektionseinrichtung einnimmt, z.B. dieselbe Lage auf dem Objekttisch eines Mikroskops. Alternativ kann die Zuordnung und Speicherung der Position des Abbilds durch Identifikation der Position einer Markierung auf dem Trägersubstrat erfolgen in Relation zum Gesichtsfeld und/oder zum Strahlengang der Detektionseinrichtung erfolgen. Auf Grundlage der durch die Immobilisierung von Zellen auf dem Trägersubstrat festgelegte Position ist eine wiederholte Detektion der selben Zelle bzw. des selben Abschnitts des Trägersubstrats mit Antikörperkonjugaten jeweils unterschiedlicher Antigenspezifität möglich, wobei jedes Antikörperkonjugat separat detektiert wird, und daher störende Wechselwirkungen mit anderen Antikörperkonjugaten reduziert bzw. vermieden werden.

Alternativ oder zusätzlich zur spezifischen Detektion von Zellbestandteilen mittels eines Antikörperkonjugats kann das erfindungsgemäße Verfahren zumindest eine Gewebsfärbung vor oder nach der Kontaktierung mit Antikörperkonjugat umfassen, z.B. die Hämatoxylin-Eosin Färbung und/oder die May-Grünwald-Färbung.

Vorzugsweise wird die ortsspezifische Zuordnung des Abbilds bei der virtuellen Überlagerung von Abbildern, die bei Kontaktierung der Probe mit verschiedenen Antikörperkonjugaten detektiert wurden, die ortsspezifische Zuordnung von Zellen mit der virtuellen Mustererkennung eines für jedes Abbild aufgenommenen lichtmikroskopischen Abbilds und die virtuelle passende Ausrichtung des erkannten Musters von Zellen zur Verbesserung der Genauigkeit kombiniert.

Weiterhin erlaubt die Wiederholbarkeit der Analyse lebender Zellen mit Antikörperkonjugaten unterschiedlicher Antigenspezifität während einer Dauer von mindestens 5 bis 10h die Auswahl von Antikörperkonjugaten auf Basis der Abbilder, die bei vorherigen Kontaktierungen der immobilisierten Zellen mit Antikörperkonjugaten anderer Antigenspezifität detektiert wurden. Dieses iterative Analyseverfahren ermöglicht die positionsabhängige Zuordnung der für jedes Antikörperkonjugat detektierten Abbilder und deren virtuelle Überlagerung. Daher ermöglicht das erfindungsgemäße Analyseverfahren durch die positionsabhängige Zuordnung der Abbilder zum einen die Analyse der selben Zellen bzw. des selben Gewebsstücks die spätere virtuelle Überlagerung der Abbilder und damit eine genaue Analyse einzelner Zellen oder Gewebsabschnitte, zum anderen die Auswahl von Antikörperkonjugaten mit einer besonderen Antigenspezifität auf Basis der Abbilder, die zuvor für einen Abschnitt des Trägersubstrats einer ausgewählten und festgelegten Position für Antikörperkonjugate detektiert wurden. Die Auswahl der Antikörperkonjugate kann ohne Abstimmung des Fluorochromanteils mit Fluorochromanteilen anderer Antikörperkonjugate erfolgen, da Wechselwirkungen der Fluorochromanteile vermieden werden, so dass die nacheinander verwendeten Antikörperkonjugate den gleichen Fluorochromanteil aufweisen können.

Besonders bevorzugt enthält das Verfahren den Schritt der Aufnahme eines lichtmikroskopischen Abbilds der Zellen, und deren automatischer Positionsbestimmung, optional gekoppelt mit dem Schritt der positionsgenauen Zuordnung der automatisch bestimmten Positionen zu detektierten Fluoreszenzsignalen. Die positionsabhängige Zuordnung der aus der Detektion von Fluoreszenzsignalen erzeugten Abbilder ist eine positionsgenau wiederholbare Detektion eines Abschnitts des Trägersubstrats bzw. der darauf immobilisieren Zellen, insbesondere bei Ausführungsformen, in denen das Trägersubstrat bei der Detektion unterschiedlicher Antikörperkonjugate die selbe Position im Gesichtsfeld der optischen Detektionseinrichtung einnimmt.

### Genaue Beschreibung der Erfindung

Die Erfindung wird nun genauer anhand von Beispielen mit Bezug auf die Figuren beschrieben, in denen
- Figur 1 unter A) als Vergleich eine gereinigte Glasoberfläche und unter B) eine mit Oligonukleinsäuren beschichtete Glasoberfläche in Einzelmoleküldetektion zeigt,
- Figur 2 die Abbilder immobilisierter Zellen nach antigenspezifischer Markierung mit Fluoreszenzdetektion unter A) als Vergleich auf einer gereinigten Glasoberfläche und unter B) auf einer mit Oligonukleinsäuren beschichteten Glasoberfläche zeigt,
- Figur 3 lichtmikroskopische Abbilder von Zellen unter A) als Vergleich auf einer gereinigten Glasoberfläche und unter B) auf einer mit Oligonukleinsäuren beschichteten Glasoberfläche zeigt,
- Figur 4 die Ergebnisse einer Vitalitätsprüfung auf einer Trägeroberfläche immobilisierter lebender menschlicher Immunzellen mit und ohne unspezifische Stimulation zeigt,
- Figur 5A) unter I) immobilisierte Leukozyten der Maus nach Inkubation mit anti-CD4-Antikörper-PE-Konjugat im mikroskopischen Bild unter Anregung der Fluoreszenz, unter II) die automatische Erkennung und Intensitätsmessung der vom Fluorochrom emittierten Strahlung, und unter III) eine grafische Darstellung der Fluoreszenzintensität (Y-Achse) über die Zellzahl (X-Achse) in Form eines Dotplots und Histogramms zeigt,
- Figur 5B) von links nach rechts mikroskopische Aufnahmen unter Fluoreszenzanregung aufeinander folgender Inkubationen, jeweils mit Antikörperkonjugaten anderer Antigenspezifität im erfindungsgemäßen Verfahren zeigt,
- Figur 6 Abbildungen der detektierten Emission von Fluorochromen nacheinander verwendeter Antikörperkonjugate mit verschiedenen Antigenspezifitäten (links neben Abbildungen angegeben) für nacheinander durchgeführte Kontaktierungen jeweils einer immobilisierten Probe (Zelle 1 bzw. Zelle 2) einschließlich Detektion intrazellulärer Antigene mit jeweils zwischenzeitlicher Inaktivierung des Fluorochromanteils zeigt,
- Figur 7 für Aliquots derselben Probe mit denselben Antikörperkonjugaten unter A) das Ergebnis einer Analyse per Durchflußzytometrie zeigt, und unter B) die Ergebnisse eines erfindungsgemäßen Verfahrens (iSBC) mit menschlichen Zellen als immobilisierter biologischer Probe,
- Figur 8 einen schematischen Aufbau einer Ausführungsform des mit Oligonukleinsäuren beschichteten Trägersubstrats zur Analyse einer Gewebsprobe zeigt und
- Figur 9 das Ergebnis der Analyse eines Gewebsschnitts zeigt.

### Beispiel 1: Herstellung eines Durchflusskanals mit Oligonukleotid - Beschichtung

Ein Kanal zur Verwendung im erfindungsgemäßen Analyseverfahren wurde durch abschnittsweises Herstellen einer kovalent gebundenen Oligonukleotid - Schicht auf einem silikatglashaltigen Trägersubstrat hergestellt. Als Trägersubstrat diente ein herkömmliches Deckglas, das auf zwei beabstandeten Abstandshaltern wurde. Die Abstandshalter waren auf einem Objektträger aus Glas fixiert, der als Deckel diente. In dem von Trägersubstrat, Abstandshaltern und Deckel aufgespannten Kanal wurde die kovalent gebundene Beschichtung aus Oligonukleotiden auf das Trägersubstrat durch oberflächliches Spülen oder Eintauchen in 1% Flusssäure in Wasser für 10 min bei Raumtemperatur, Entfernen der Flusssäure durch Spülen mit Aceton, Entfernen des Acetons und Kontaktieren mit einer methanolischen Lösung tri-Methoxysilangruppen-haltiger Oligonukleinsäuren (dT₃₅) hergestellt. Überschüssiges Oligonukleotid konnte durch Waschen in PBS (phosphatgepufferte Salzlösung, pH 7,4) entfernt werden. Die tri-Methoxysilangruppe war in der folgenden Verbindung an die 35-dT-Oligonukleinsäure (Oligo) gekoppelt:

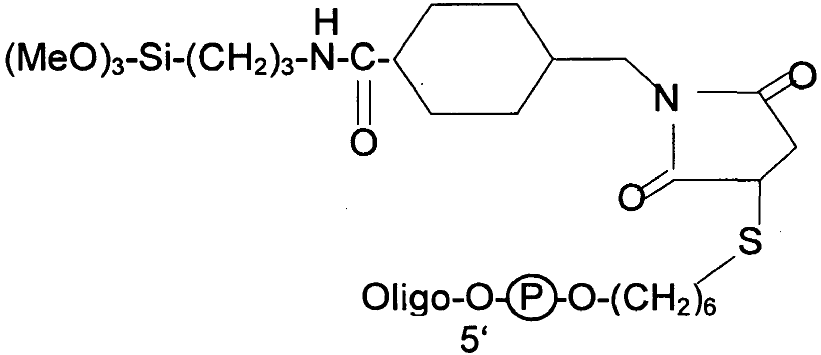

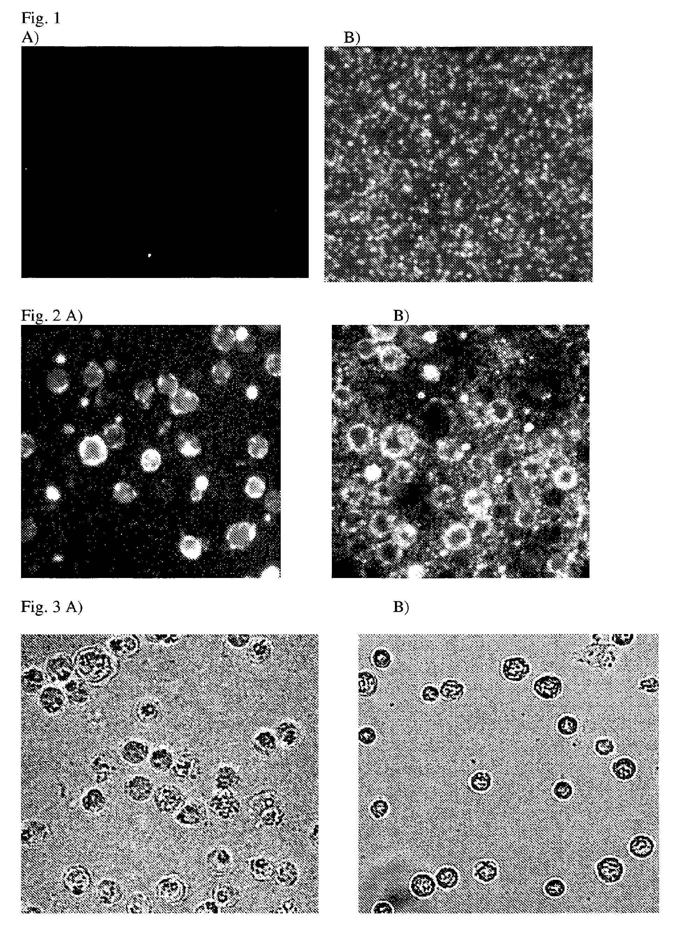

Entsprechend kann das Oligo anstelle des 35-dT-mers ein anderes Oligonukleotid synthetischer oder natürlicher Herkunft mit dieser oder einer anderen Kopplungsgruppe mit einer Alkoxysilangruppe verbunden sein. Als Test für die Verteilung und Bindung der Oligonukleinsäuremoleküle an die Glasoberfläche des Trägersubstrats wurde eine mit Flusssäure geätzte Glasoberfläche als Vergleich zur erfindungsgemäß mit der Oligo-dT-Nukleinsäure mit dem Fluoreszenzfarbstoff-markierten dA₂₆-Cy3 in hoher Verdünnung kontaktiert. Das Ergebnis der fluoreszenzmikroskopischen Analyse (Axioplan 2e Mikroskop, 100x Plan-Achromat Objektiv zur Einzelmoleküldetektion) ist in Figur 1 gezeigt, unter A) die gereinigte Glasoberfläche zum Vergleich, unter B) die mit kovalent gebundenem dT₃₅ beschichtete Glasoberfläche. Diese Analyse zeigt, dass die Fluoreszenzmarkierung auf die kovalent gebundene Oligonukleinsäurebeschichtung zurückgeht und die Oligonukleinsäuremoleküle gleichmäßig über die Fläche verteilt sind.

Die Nukleinsäurebeschichtung war so vollständig bzw. kontinuierlich, dass die kontaktierte Oberfläche bei Kontaktierung mit einer Zellsuspension, z.B. mit Ficoll-Gradienten isolierte Leukozyten, die Zellen zu einer einschichtigen Zellschicht immobilisierte, wobei die Dichte immobilisierter Zellen abhängig von der Zellzahl war. Für die Einzelzellanalyse ist es bevorzugt, dass die Zellen beabstandet auf dem Trägersubstrat immobilisiert sind, um die Wiedererkennung der Einzelzellen anhand ihrer Position relativ zum Trägersubstrat zu erleichtern.

Die Immobilisierung von Zellen auf dem Oligonukleinsäure-beschichteten silikathaltigen Trägersubstrat erfolgt durch Kontaktieren des Oligonukleinsäure-beschichteten silikathaltigen Trägersubstrats mit den Zellen, die z.B. in Suspension vorliegen. Zur Kontaktierung ist das Aufgeben der Zellen in wässriger Zusammensetzung auf das Trägersubstrat bei 20 bis 36°C ausreichend, z.B. durch Pipettieren der Zellsuspension in die Einlassöffnung des die Beschichtung des Trägersubstrats umfassenden Kanals.

Zur optischen Analyse wurde der Objektträger auf dem Objekttisch eines Mikroskops platziert, so dass das mit Oligonukleinsäuren beschichtete Deckglas oberhalb des Objektträgers angeordnet war. Daher war das mit Oligonukleinsäuren beschichtete Trägersubstrat oberhalb des vom Objektträger gebildeten Deckels und oberhalb des Kanalvolumens angeordnet.

Die Verteilung der immobilisierten Zellen auf dem mit Oligonukleinsäure beschichteten Deckglas zeigt, dass die Oligonukleinsäure-Beschichtung generell eine gleichmäßige Immobilisierung von Zellen ergibt. Die Immobilisierungskapazität von ca. 2000 bis 2500 Zellen/0,15µm² (Gesichtsfeld) ist signifikant höher als die von Trägersubstraten mit immobilisiertem Fängerantikörper. Das geringe Kanalvolumen oberhalb des Detektionsabschnitts von ca. 2µL erlaubt überdies die Verwendung für kleine Volumina an Probe und an Antikörperkonjugat. So reichen für ein Innenvolumen des Kanals, das an den mit Oligonukleinsäuren beschichteten Detektionsabschnitt angrenzt, ca. 10.000 bis 100.000 Zellen in einem Volumen von ca. 2 µL, um eine derzeit als optimal angesehene Dichte immobilisierter Zellen von ca. 400 bis 1800 Zellen/0,15µm² zu erhalten.

Weiterhin zeigt ein Vergleich der Analyse eines Aliquots von Zellen, das mit einer mit Oligonukleinsäuren beschichteten Glasoberfläche kontaktiert wurde, zu einem Aliquot, das mit einer durch Ätzen in Flusssäure gereinigten Glasoberfläche kontaktiert wurde, dass das die erfindungsgemäße Beschichtung mit Oligonukleinsäuren unspezifische Signale bei Fluoreszenzdetektion verringert, bzw. eine empfindlichere Detektion von fluoreszent markierten Analyten erlaubt. Fluoreszenzmikroskopische Aufnahmen sind in Figur 2A) für die gereinigte Glasoberfläche und in 2B) für die mit dT₃₅ beschichtete Glasoberfläche nach Kontaktierung mit menschlichen PBMC (einkernige Zellen aus Blut) und Kontaktieren mit 10 µg/mL anti-CXCR5-PE-Antikörperkonjugat für 5min gezeigt. Es wird deutlich, dass die Beschichtung mit Oligonukleinsäure eine signifikant bessere Detektion der spezifisch markierten Zellen ergibt, während unspezifische Hintergrundsignale durch die Beschichtung mit Oligonukleinsäure signifikant verringert sind.

Die lichtmikroskopische Analyse zeigt die besondere Eignung der Beschichtung mit Oligonukleinsäure zur Verwendung für die Fixierung permeabilisierter eukaryotischer Zellen. In Figur 3A) sind PBMC auf der durch Ätzen mit Flusssäure gereinigten Glasoberfläche gezeigt, in 3B) die PBMC auf der mit dT₃₅ beschichteten Glasoberfläche, wobei die Zellen jeweils nach Kontaktierung mit der Oberfläche durch Saponin-haltigen Puffer (FixPerm, BD Biosciences) fixiert und permeabilisiert wurden. Die Zellkonturen der auf mit Oligonukleinsäure beschichteten Glasoberfläche sind deutlich besser erhalten, als die Konturen der Zellen auf der gereinigten Glasoberfläche. Dies zeigt, dass die Beschichtung mit Oligonukleinsäure die Zellstrukturen bei Fixierung und Permeabilisierung besser erhält, als eine nicht beschichtete Oberfläche.

Bei der Analyse der mit Oligonukleinsäuren versehenen Glasoberfläche hat sich gezeigt, dass bei Verringerung der Konzentration des im Herstellungsverfahren eingesetzten dT35-Methoxysilan-Oligomers zu einer nicht ausreichend dichten Bindung von Oligonukleinsäuren führt. So wurde durch Einzelmolekül-Detektion von dA26-Cy3 an immobilisiertes oligo-dT35 gefunden, dass bei Konzentrationen von 0,0017pm/µL dT35-Methoxysilan-Oligomer 14 Moleküle oligo-dT35/100µm² der Glasoberfläche, bei 0,017 pm/µL dT35-Methoxysilan-Oligomer 187 Moleküle oligo-dT35/100µm² der Glasoberfläche und bei 0,17 pm/µL dT35-Methoxysilan-Oligomer >5000 Moleküle oligo-dT35/100µm² Glasoberfläche gebunden waren, was der Auflösung des Mikroskops bei der verwendeten Fluororeszenzdetektion entspricht bzw. diese überschreitet. Für eine effiziente und zelltypunspezifische Bindung von Zellen an das mit Oligonukleinsäuren versehene Trägersubstrat weist dieses daher generell vorzugsweise eine Dichte von zumindest 5000 Molekülen Oligonukleinsäuren pro 100µm² Oberfläche auf, bevorzugt eine Dichte zumindest 10.000 Molekülen Oligonukleinsäuren pro 100µm² Oberfläche.

### Beispiel 2: Immobilisierung und Analyse lebender eukaryotischer Zellen

Das erfindungsgemäße Analyseverfahren eignet sich sowohl für immobilisierte lebende Zellen, als auch für immobilisierte Zellen, die nach Kontaktierung mit der mit Oligonukleinsäuren beschichteten Silikatoberfläche in herkömmlicher Weise denaturiert und/oder perforiert sind, beispielsweise durch Inkubation mit Formalin, Aceton, Methanol und/oder Saponin.

Als Beispiel für eukaryotische Zellen wurden lebende menschliche Immunzellen verwendet, die nach Abtrennung von Erythrozyten in Suspension in PBS vorlagen. Zur Immobilisierung der Zellen auf dem mit Oligonukleotiden beschichteten Trägersubstrat wurden diese in einen Kanal einströmen gelassen, dessen eine Innenseite von dem mit Oligonukleinsäuren beschichteten Trägersubstrat gebildet wurde.

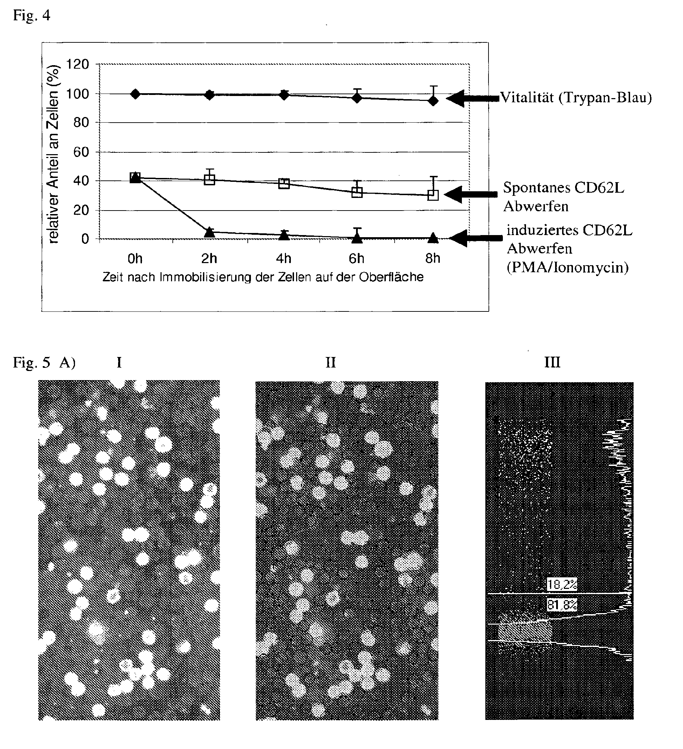

Figur 4 zeigt die funktionelle Analyse von lebenden menschlichen Immunzellen, die auf der Oberfläche eines nach Beispiel 1 mit Oligonukleinsäuren beschichteten Deckglases immobilisiert sind. Die Lebendfärbung mit 10 µL Trypanblau zeigt, dass zumindest über eine Immobilisierung für eine Dauer von 8 Stunden die Anzahl lebender Zellen nur geringfügig abnimmt. Das Abwerfen des CD62L von der Zelloberfläche (offene Kästen) ohne Zusatz eines Stimulanzes, d.h. spontan, zeigt zumindest über 8 h, vorzugsweise über 2 bis 4 h, eine nur geringfügige Beeinträchtigung der Zellen durch die Immobilisierung, im Unterschied zur Aktivierung durch Zusatz von PMA/Ionomycin, die zu einem Abwerfen von CD62L innerhalb der ersten zwei Stunden der Fixierung führte.

Entsprechend ist bevorzugt, das Verfahren zur Analyse zunächst an lebenden, immobilisierten biologischen Proben, insbesondere eukaryotischen Zellen durchzuführen, und diese Zellen im Anschluss zu denaturieren und/oder zu perforieren, um anschließend an den denaturierten bzw. fixierten Zellen das Analyseverfahren durchzuführen.

### Beispiel 3: Detektion oberflächlicher Antigene in immobilisierten lebenden eurkaryotischen Zellen mit automatischer Positionsbestimmung der Zellen

Leukozyten aus peripherem Blut der Maus wurden nach Erythrolyse durch Zentrifugation von Erythrozyten-Resten abgetrennt und im Serum auf ein nach Beispiel 1 hergestelltes Trägersubstrat pipettiert, das von einem um 20 µm beabstandeten Objektträger als Deckel überdeckt war. Nach Inkubation bei Raumtemperatur für 5 min in der Waagerechten wurde der Überstand durch Zugabe von PBS (pH 7,4) verdrängt.

Das Trägersubstrat wurde in den Strahlengang eines Mikroskops (Axioplan 2e Mikroskop, Zeiss, mit motorischer Objekttischverstellung und Fokussierung, Quecksilberlampe HBO 100 zur Anregung, Filter für PE oder FITC, Plan-Neofluar 16x/0,50 Immersionsobjektiv und CCD Kamera Axiocam MRm zur Aufzeichnung, in allen Beispielen verwendet) positioniert, nachdem oder bevor PE-konjugierte anti-CD4-Antikörper (10 µg/mL, 10 µL) in PBS zugegeben wurden. Entsprechend der bevorzugten Ausführungsform war das als Trägersubstrat verwendete Deckglas waagerecht und oberhalb des Objektträgers (Deckel) im Gesichtsfeld des Mikroskops angeordnet, so dass der mit Oligonukleinsäuren beschichtete Detektionsabschnitt die obere Wandung des Kanals zwischen Trägersubstrat und beabstandetem Deckel bildete. In dieser Position hängen die Zellen in das Innenvolumen des Kanals, und es wurde gefunden, dass so die Immobilisierung die Morphologie lebender Zellen nur geringfügig beeinflusst.

Nach Inkubation bei Raumtemperatur für 5 min wurde ungebundener Antikörper durch Waschen der immobilisierten Zellen mittels Aufgeben von 100 µL PBS an einem Ende des Deckglases und Absaugen austretender Flüssigkeit am gegenüberliegenden Ende entfernt. Die Detektion gebundenen Antikörpers erfolgte mikroskopisch durch Einstrahlung mit einer Wellenlänge von 488 nm. Das mikroskopische Bild mit Fluoreszenzanregung ist in Figur 5 A) I gezeigt, die bevorzugt durchzuführende automatische Bilderkennung in II gezeigt, und in III ist die durch die automatische Bilderkennung vorgenommene Zuordnung jeder Zelle zu einer bestimmten Position des Detektionsabschnitts, und in Verbindung damit zur automatisch bestimmten Position des Trägersubstrats gezeigt, was eine definierte Zuordnung jeder Zelle zu einer räumlichen Position relativ zum Trägersubstrat erlaubt, und daher ohne Verschiebung des Trägersubstrats und auch nach Entfernung und erneutem Anordnen des Trägersubstrats im Gesichtsfeld des Mikroskops eine eindeutige Identifikation und Zuordnung jeder Zelle auf Grundlage der automatisch bestimmten Position auf dem Trägersubstrat.

Die automatische Positionsbestimmung von Zellen im Gesichtsfeld des Mikroskops erfolgte mittels eines selbst entwickelten Programms, mit zusätzlicher automatischer Zuordnung der Position des rechnergesteuert verfahrbaren Objekttisches (Merzheuser, Deutschland) zu jeder bestimmten Position einer Zelle. Die Aufnahmen wurden durch Detektion für eine Dauer von 7 s mit Fluoreszenzdetektion (Durchlicht 100 ms) gemacht. Bei Entnahme des Trägersubstrats vom Objekttisch, z.B. zur weiteren Zugabe und/oder Entfernung von Lösungen zum Waschen und mit weiteren Antikörpern, wurde die Position der Zellen auf dem Trägersubstrat nicht verändert, so dass allein durch Korrelation der Positionierung des Trägersubstrats bzw. des diesen in der selben Position halternden Objekttisches jede Zelle positionsgenau erneut detektiert werden konnte, und mit verschiedenen Antikörpern nacheinander aufgenommene mikroskopische Fluoreszenzbilder für jede Zelle spezifisch überlagert werden konnten. Die in Figur 5 A) II eingefügten Kreise stehen für Positionen der nicht vom Antikörper markierten Zellen, die in einer vorherigen lichtmikroskopischen Aufnahme identifiziert wurden. Figur III zeigt die Verteilung der Intensität der für alle Zellen gemessenen Fluoreszenz über die Zellzahl.

### Beispiel 4: Nacheinander folgende Detektion verschiedener oberflächlicher Antigene in immobilisierten lebenden eukaryotischen Zellen

Zellen aus einer broncho-alveolären Spülung einer Maus mit induziertem Asthma (entsprechend Polte et al., J. Allergy Clin. Immunol. 118, 942-8 (2006)) wurden gemäß Beispiel 2 immobilisiert. Die immobilisierten Zellen wurden nacheinander mit Antikörperkonjugaten, die jeweils das Fluorochrom PE enthielten, kontaktiert, jeweils mit Unwirksammachen des Fluorochroms vor Zugabe eines neuen Antikörperkonjugats, z.B. mittels Bestrahlung bei der für das Fluorochrom spezifischen Anregungswellenlänge (488 nm) für ca. 30 s, mit Waschen nach Zugabe eines neuen Antikörpers und Detektion unter Fluoreszenzanregung. Die Antikörperkonjugate unterschieden sich nur durch ihren Antikörperanteil, der eine Spezifität für CD11b, B7H 1, CD11c bzw. CD3 hatte.

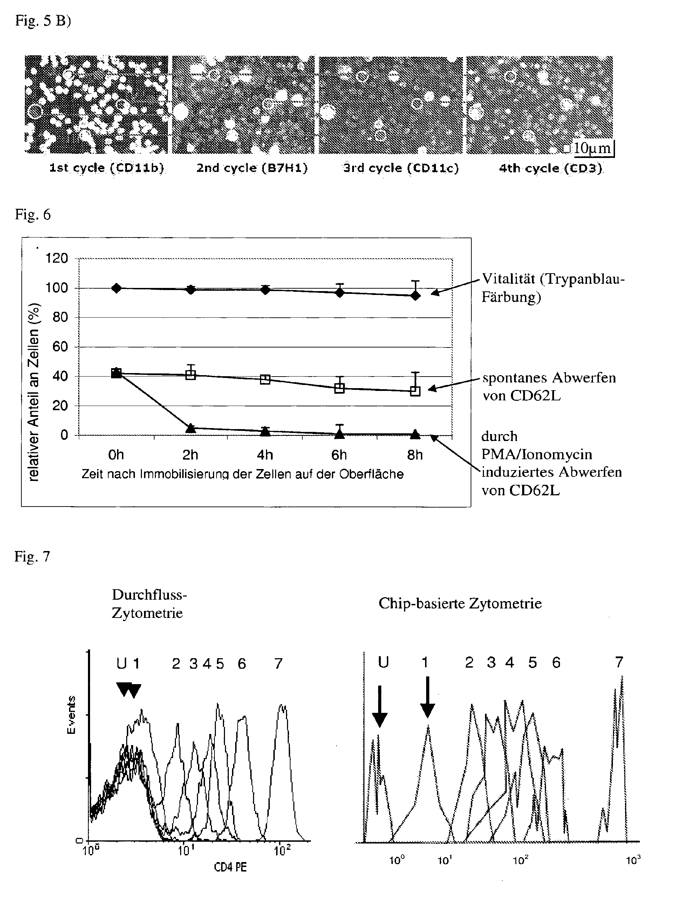

Figur 5B) zeigt von links nach rechts angeordnet mikroskopische Aufnahmen mit Detektion emittierter Fluoreszenz; die Spezifitäten der Antikörper, nämlich anti-CD11b, anti-B7H1, anti-CD11c und anti-CD3, sind unter den jeweiligen Aufnahmen angegeben. Die grafische Markierung einzelner Lokalisationen zeigt, dass die erfindungsgemäße Zuordnung jeweils für ein Antikörperkonjugat spezifisch detektierter Fluoreszenz zu einer Position des mikroskopischen Abbilds die Überlagerung der aufeinander folgend detektierten Fluoreszenzsignale erlaubt. In der Folge erlaubt das erfindungsgemäße Verfahren die Detektion mehrerer zu analysierender Antigene in immobilisierten biologischen Proben nacheinander, jeweils mit Antikörperkonjugaten mit Spezifität für die zu analysierenden Antigene, mit demselben Fluorochromanteil oder unterschiedlichen Fluorochromanteilen. Diese Analyse zeigt, dass einzelne Positionen einer biologischen Probe, z.B. einzelne Zellen, nacheinander auf eine Mehrzahl von Antigenen analysiert werden können, ohne dass Beeinträchtigungen der Analyse durch Wechselwirkungen von Antikörperkonjugaten oder deren Fluorochromen auftreten, oder unspezifische Fluoreszenz entsteht, wobei die Oligonukleinsäure-beschichtete Oberfläche des Trägersubstrats eine ausreichend lange Immobilisierung der Zellen im lebenden Zustand erlaubt. Auf Basis der Immobilisierung ist die automatische Positionsbestimmung der lichtmikroskopisch analysierten Position jeder Zelle und der detektierten Fluoreszenzsignale möglich, und die anschließende positionsgenaue Überlagerung bzw. Korrelation der lichtmikroskopischen Aufnahme und/oder von detektierten Fluoreszenzsignalen.

Die Überlagerung jeweils gleicher Positionen nachgewiesener Fluoreszenz, vorzugsweise in Verbindung mit der lichtmikroskopisch bestimmten Position von Zellen, erlaubt die in getrennten Schritten aufeinander folgende Detektion verschiedener Antigene und deren anschließende Zuordnung zu einer Zelle. Diese positionsgenaue Korrelation der detektierten Fluoreszenzsignale ist in Figur 5 B) durch die eingefügten Verbindungslinien zwischen eingekreisten Zellen dargestellt.

### Beispiel 5: Nacheinander folgende Detektion intrazellulärer und oberflächlicher Antigene in fixierten und permeabilisierten eukaryotischen Zellen

Als Beispiel für eine biologische Probe wurden menschliche Leukozyten entsprechend Beispiel 2 immobilisiert, anschließend jedoch mit Formaldehyd fixiert und mit Saponinhaltigem Puffer (FixPerm, BD Biosciences) permeabilisiert.

Die auf dem mit Oligonukleinsäuren beschichteten Trägersubstrat fixierten Zellen wurden mit 10µL (2µg/mL - 200ng/mL) Antikörperkonjugat für 5 min bei Raumtemperatur kontaktiert und mit 200µL PBS gewaschen. Generell wurde nach der Kontaktierung mit einem Antikörperkonjugat die unter Bestrahlung mit Licht der Anregungswellenlänge emittierte Strahlung detektiert, anschließend der Fluorochromanteil des Antikörperkonjugats durch Bestrahlung bei der für das Fluorochrom spezifischen Wellenlänge (488 nm) unwirksam gemacht, und anschließend wurde ein anderes Antikörperkonjugat mit der Probe kontaktiert.

Das als Trägersubstrat dienende, mit Oligonukleinsäuren beschichtete Deckglas war oberhalb eines Objektträgers angeordnet, der durch zwei stegförmige Abstandshalter von ca. 150 µm Dicke von dem Trägersubstrat beabstandet war, sodass die Stege den Raum zwischen Trägersubstrat und beabstandetem Objektträger zu einem Durchflusskanal begrenzten. Die Schritte der Kontaktierung der Probe mit Antikörper-Konjugat und die Waschschritte nach Zugabe eines anderen Antikörperkonjugats konnten jeweils durch Pipettieren der entsprechenden wässrigen Zusammensetzung an ein Ende des Durchflusskanals erfolgen, wahlweise in Verbindung mit Absaugen von Flüssigkeit am gegenüberliegenden Ende des Durchflusskanals.

Nach Inkubation der immobilisierten Zellen mit dem anti-IFNγ-Antikörperkonjugat für 5 min bei Raumtemperatur und einem anschließenden Waschschritt wurde die Fluoreszenz mikroskopisch detektiert. Die detektierte Fluoreszenz einschließlich des mikroskopischen Abbilds wurden unter Zuordnung zur Positionierung des Trägersubstrats, bzw. bei unveränderter Positionierung des Trägersubstrats durch Speichern in einem elektronischen Speicher aufgezeichnet. Anschließend wurde der Fluorochromanteil durch Bestrahlung bei 488 nm ausgeblichen.

Im Anschluss an dieses Unwirksammachen des Fluorochromanteils des ersten Antikörperkonjugats mit Spezifität für IFN-γ wurde ein Antikörperkonjugat mit der Probe kontaktiert, das identisch aufgebaut war, außer dass der Antikörperanteil nun ein anti-CD3-Antikörper war. Anschließend wurde wiederum Bestrahlung 488 nm der Fluorochromanteil des Antikörperkonjugats mit anti-CD3-Spezifität unwirksam gemacht.

In der selben Art und Weise wurde anschließend dieselbe Probe nacheinander mit Antikörperkonjugaten mit einem anti-CD4-Antikörperanteil, einem anti-CD8-Antikörperanteil bzw. einem anti-IL4 - Antikörperanteil inkubiert, jeweils mit Unwirksammachen des Fluorochromanteils vor der Inkubation mit einem Antikörper mit anderer (zusätzlicher) Antigenspezifität, jeweils mit einem Waschschritt im Anschluss an die Zugabe eines Antikörperkonjugats zur Entfernung ungebundener Fluorochrome. Mikroskopische Abbildungen sind in Figur 6 für Zelle 1 und Zelle 2 gezeigt, wobei im Wesentlichen derselbe Bildausschnitt, der durch den manuell eingefügten Kreis gekennzeichnet ist, detektiert wurde. Es wird deutlich, dass das Unwirksammachen des Fluorochromanteils der jeweiligen Antikörperkonjugate zu einer Reduktion bzw. Eliminierung der Emissionen des zuvor verwendeten Antikörperkonjugats führte, jedenfalls auf ein Niveau unterhalb der Detektionsgrenze, wobei auch in folgenden Zyklen des Analyseverfahrens mit Antikörperkonjugaten anderer Antigenspezifität keine zusätzliche Hintergrundaktivität bzw. Hintergrund-Fluoreszenz auftrat. Weiterhin zeigt dieses Beispiel, dass sowohl die extrazellulären Antigene CD3, CD4, CD8, als auch die intrazellulären Antigene IFN-γ und der IL4 unabhängig voneinander in den fixierten Zellen nachgewiesen werden konnten.

### Beispiel 6: Vergleich der Spezifität und der Empfindlichkeit des erfindungsgemäßen Verfahrens gegenüber Durchflußzytometrie

Das erfindungsgemäße Analyseverfahren wurde an menschlichen Leukozyten, die auf einem Trägersubstrat immobilisiert waren, und zum Vergleich durch herkömmliche Durchflußzytometrie-Analyse analysiert. Aliquots von Milzzellen der Maus (5 Tiere) wurden mit Antikörperkonjugaten verschiedener Antigenspezifität kontaktiert, um die Identifikation der jeweiligen Antigene auf den Zellen im Vergleich zu prüfen. Für die Vergleichsanalyse mittels Durchflußzytometrie (FACScan, Becton Dickinson) wurden Aliquots der Zellsuspensionen in 50 µL PBS mit 1% RSA und mit jeweils einem der folgenden Antikörperkonjugate (1µL, 0,2 µg/mL) inkubiert: anti-CD3-PerCP, anti-CD4-PE, anti-CD8-FITC, anti-CD 19-PerCP, anti-CD4-PE bzw. anti-CD3-FITC und für Antikörperkonjugate mit gleichem Fluorochromanteil separat analysiert.

Für das erfindungsgemäße Analyseverfahren wurden Leukozyten auf einem mit Oligonukleotiden beschichteten Trägersubstrat immobilisiert, der in einem Durchflusskanal angeordnet war und nacheinander mit den folgenden Antikörperkonjugaten kontaktiert, jeweils mit zwischenzeitlicher Inaktivierung des Fluorochromanteils durch UV-Bestrahlung: anti-CD3-PE, anti-CD4-PE, anti-CD8-PE, anti-CD19-PE, ebenfalls in 50 µL PBS mit 1% RSA und 1 µL, 0,2 µg/mL Antikörperkonjugat.

Die folgenden Anteile von Zellen wurden mit den Antikörperkonjugaten bestimmt:

| Antigen | Durchflußzytometrie | erfindungsgemäß an immobilisierten Zellen |
|---|---|---|
| CD 19+ | 60% +/-1 | 60% +/-0,7 |
| CD 19+ CD4+ | 0% | 0% |
| CD3+ | 39% +/-2,5 | 40% +/-3 |
| CD4+ | 24,5% +/-2 | 27% +/-1,5 |
| CD8+ | 15 % +/-2 | 14,5% +/-1,5 |
| CD4+ CD3+ | 17% +/-3 | 19% +/-2 |

Diese Ergebnisse zeigen, dass mit beiden Verfahren dieselben Antikörper-spezifischen Zellpopulationen in den Proben identifiziert wurden. Daher hat das erfindungsgemäße Verfahren im wesentlichen dieselbe Spezifität wie die Analyse per Durchflusszytometrie (FACS).

Die Nachweisempfindlichkeit des erfindungsgemäßen Verfahrens wurde ebenfalls im Vergleich zu FACS geprüft, indem nach Beispiel 2 Milzzellen der Maus auf einem mit Oligonukleotiden beschichteten Deckglas immobilisiert wurden und nacheinander mit abnehmenden Verdünnungen, d.h. zunehmenden Konzentrationen von anti-CD4-PE Antikörperkonjugat kontaktiert wurden, mit Inaktivierung des Fluorochroms PE nach jeder Detektion. Für die Vergleichsanalyse per FACS wurden 7 Aliquots der Zellsuspension mit denselben Verdünnungen des anti-CD4-PE inkubiert und analysiert.

Die Ergebnisse sind zusammengefasst grafisch in Figur 7A) für Durchflußzytometrie und 7 B) für das erfindungsgemäße Analyseverfahren gezeigt, wobei Spitzenwerte mit Hinweisen auf die Konzentration des Antikörpers gekennzeichnet sind mit U = Negativkontrolle ohne Antikörperkonjugat und 1 bis 7 für zunehmende Konzentration. Die Ergebnisse zeigen, dass die Empfindlichkeit des erfindungsgemäßen Analyseverfahrens etwa um den Faktor 10 höher als bei Durchflußzytometrie ist. So zeigt sich bei 1, der geringsten Antikörperkonzentration, im Durchflußzytometrie keine Auflösung vom Hintergrund (U), während das erfindungsgemäße Verfahren bereits für diese Antikörperkonzentration ein deutlich vom Hintergrund unterscheidbares Signal detektierbar macht.

### Beispiel 7: Nachweis von Makrophagen in Lungengewebe

Als Beispiel für den Nachweis von zellspezifischen Oberflächen-Antigenen in einem Gewebsstück mit lebenden Zellen wurde ein Gewebsschnitt mittels vibrierendem Mikrotom (erhältlich von der Fa. Vibratom) aus Lungengewebe der Maus auf einem Trägersubstrat immobilisiert. Das Lungengewebe lebte und war dadurch immobilisiert, dass das Trägersubstrat einen formschlüssigen Rahmen aus elastischem Material aufwies, der durch Ausschneiden einer dem Gewebsschnitt entsprechenden Form aus Kunststofffolie und Anordnen dieses Rahmens auf einem Deckglas. Als Kunststofffolie konnte Polyethylen, Polypropylen und vorzugsweise Parafilm verwendet werden. Die Kunststofffolie hatte vorzugsweise eine Dicke von ca. 200 µm; der Gewebsschnitt hatte eine Schichtdicke von ca. 100 µm. Das als Trägersubstrat verwendete Deckglas war in dem vom Rahmen umgrenzten Abschnitt entsprechend Beispiel 1 zusätzlich mit Oligonukleinsäuren beschichtet. Als Deckel wurde ein Objektträger aus Glas gegenüber dem Deckglas angeordnet. Der zum Gewebsstück formschlüssige Rahmen bildete die Abstandshalter zwischen Deckglas und Objektträger, wobei der Rahmen zwei gegenüberliegende Öffnungen durch streifenförmige Ausnehmungen im Material des Rahmens aufwies, die gegenüberliegend in das vom Rahmen umgrenzte Volumen zwischen Trägersubstrat (Deckglas) und Deckel (Objektträger) münden. Die streifenförmigen Ausnehmungen im Material des Rahmens wurden in den Abstandshaltern gebildet und stellten die Einlassöffnung und gegenüberliegend die Auslassöffnung im vom Rahmen umgrenzten Volumen dar, und damit die Einlass- und Auslassöffnungen zum darin angeordneten Gewebsstück.

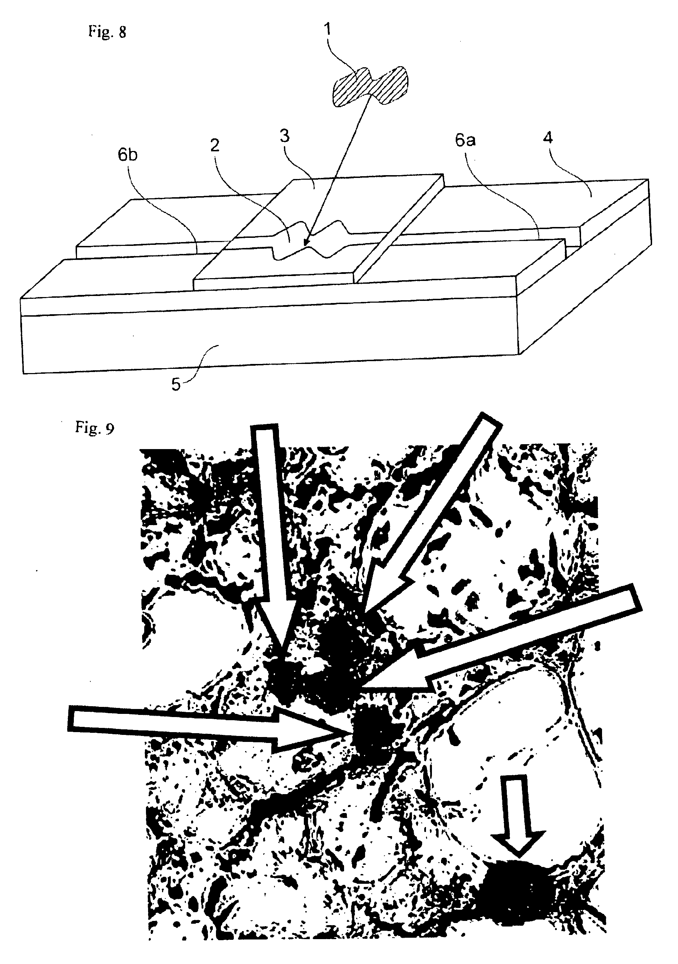

Eine schematische Ansicht des zum Gewebsstück formschlüssigen Rahmens ist in Figur 8 gezeigt:
Das Gewebsstück 1, z.B. ein Gewebsschnitt, ist in einem formschlüssigen Rahmen 2 angeordnet. Der formschlüssige Rahmen 2 ist zwischen einem Trägersubstrat 3, das z.B. ein Deckglas sein kann, und einem Deckel 5 angeordnet, der z.B. ein Objektträger sein kann. Das Trägersubstrat ist vorzugsweise zumindest in dem Abschnitt, der vom Rahmen 2 umfasst wird, mit kovalent gebundenen Oligonukleotiden beschichtet. Der Rahmen 2 ist als Ausnehmung von Abstandshaltern 4 gebildet, die zwischen Trägersubstrat 3 und Deckel 5 angeordnet sind, wobei Trägersubstrat 3 und Deckel 5 das vom Rahmen 2 aufgespannte Volumen begrenzen. In Abstandshaltern 4 sind streifen- oder nutförmige Ausnehmungen 6a, 6b gebildet, die eine Einlassöffnung und eine etwa gegenüberliegende Auslassöffnung in dem vom Rahmen 2 aufgespannten Volumen zwischen Trägersubstrat 3 und Deckel 5 bilden.

Eine mikroskopisches Abbild des mit anti-Maus-CD11b-Antikörper-PE-Konjugat entsprechend Beispiel 2 analysierten Gewebsschnitts ist in Figur 9 gezeigt. Die mit Pfeilen markierten Zellen zeigten die identifizierten CD11b+-Zellen im Gewebe und machen deutlich, dass der erfindungsgemäße, zu einem Gewebsstück formschlüssige Rahmen zwischen Trägersubstrat und Deckel zur Immobilisierung lebenden Gewebes und zur Analyse zellspezifischer Antigene im Gewebsstück geeignet ist.

Der Fluorochromanteil PE wurde durch Bestrahlung bei 488 nm ausgebleicht. Anschließend konnten in aufeinander folgenden Zyklen 30 weitere Antikörperkonjugate mit dem Gewebsstück kontaktiert werden. Die Antikörperkonjugate wiesen PE als Fluorochromanteil auf und einen jeweils unterschiedlichen Antikörperanteil. Das Trägersubstrat war für jede Detektion in der selben Position im Strahlengang des als Detektionseinrichtung verwendeten Mikroskops (Zeiss Axioplan 2e aus Beispiel 3) angeordnet. Jeder Zyklus umfasste das Kontaktieren des Gewebsstücks mit einem Antikörperkonjugat, Waschen zur Entfernung ungebundenen Antikörperkonjugats, Detektion des vom Fluorochromanteil unter Bestrahlung mit Licht bei Anregungswellenlänge (488 nm) emittierten Lichts, Speichern des detektierten Abbilds und das Unwirksammachen des Fluorochroms, z.B. durch Ausbleichen mittels Bestrahlung bei der Anregungswellenlänge. Zur Auswertung wurden die Abbilder aus dem Speicher abgerufen und virtuell positionsgenau übereinandergelegt, da die einzelnen Abbilder jedes Antikörperkonjugats bei identischer Positionierung des Trägersubstrats in der Detektionseinrichtung detektiert wurden. Diese Verfahrensschritte der Analyse von Zellen in einem Gewebsstück sind das generell bevorzugte Verfahren.

## Patentansprüche

1. Verfahren zur Analyse eukaryotischer Zellen mit den Schritten der
Kontaktierung von Zellen oder Gewebsstücken mit einem Abschnitt eines Trägersubstrats, der in einem Kanal mit einer Einlassöffnung und einer Auslassöffnung angeordnet ist,
Kontaktierung der auf dem Abschnitt des Trägersubstrats angeordneten Zellen mit einem ersten Nachweiskonjugat, das eine erste Antigenspezifität und einen Fluorochromanteil hat,
Einstrahlen von Licht einer Anregungswellenlänge für den Fluorochromanteil, Detektieren der vom Fluorochromanteil emittierten Strahlung,
Speichern des Abbilds der detektierten Strahlung,
**dadurch gekennzeichnet, dass**
der Abschnitt des Trägersubstrats mit Oligonukleinsäuren beschichtet ist, die eine Abfolge von zumindest 90% Nukleotiden aufweisen, die jeweils dasselbe von A (Adenin), T (Thymin), G (Guanin), C (Cytosin), I (Inosin) oder U (Uridin) aufweisen und die im wässrigen Medium eine lineare Konformation aufweisen und mit genau einem ihrer Enden an das Trägersubstrat gebunden sind.

2. Verfahren nach Anspruch 1, **dadurch gekennzeichnet, dass** die Oligonukleinsäuren zu zumindest 5.000 Molekülen pro µm² in dem Abschnitt des Trägersubstrats vorliegen.

3. Verfahren nach Anspruch 1 oder 2, **dadurch gekennzeichnet, dass** die Oligonukleinsäuren einsträngig sind.

4. Verfahren nach Anspruch 1 oder 2, **dadurch gekennzeichnet, dass** die Oligonukleinsäuren eine doppelsträngige Konformation haben und aneinandergrenzende Abschnitte aufweisen, von denen ein erster Abschnitt aus oligo-A und/oder oligo-dA besteht, und ein zweiter Abschnitt aus oligo-T und/oder oligo-dT.

5. Verfahren nach einem der voranstehenden Ansprüche, **dadurch gekennzeichnet, dass** die Oligonukleinsäuren durch Kopplungsgruppen an das Trägersubstrat gebunden sind.

6. Verfahren nach einem der voranstehenden Ansprüche, **dadurch gekennzeichnet, dass** das Trägersubstrat zusätzlich zu der Beschichtung mit Oligonukleinsäuren einen zu einer Gewebsprobe formschlüssigen Rahmen aufweist, der sich bis zu einem vom Trägersubstrat beabstandeten Deckel erstreckt, wobei in dem Rahmen ein Gewebsstück angeordnet ist.

7. Verfahren nach einem der voranstehenden Ansprüche, **dadurch gekennzeichnet, dass** im Anschluss an die Detektion der vom Fluorochromanteil emittierten Strahlung das Unwirksammachen der optischen Aktivität des Fluorochromanteils erfolgt, und wiederholt die
Kontaktierung der auf dem Abschnitt des Trägersubstrats angeordneten Zellen mit einem weiteren Nachweiskonjugat, das eine weitere Antigenspezifität und einen Fluorochromanteil hat,
das Einstrahlen von Licht einer Anregungswellenlänge für den Fluorochromanteil, das Detektieren der vom Fluorochromanteil emittierten Strahlung,
das Speichern des Abbilds der jeweils detektierten Strahlung und das Unwirksammachen des Fluorochromanteils des weiteren Nachweiskonjugats erfolgt, wobei für jeweils eine Kontaktierung mit einem Nachweiskonjugat gespeicherte Abbilder virtuell positionsgenau übereinandergelagert dargestellt werden.

8. Verfahren nach Anspruch 7, **dadurch gekennzeichnet, dass** das Unwirksammachen des Fluorochromanteils durch Bestrahlung erfolgt.

9. Verfahren nach einem der Ansprüche 7 bis 8, **dadurch gekennzeichnet, dass** die Fluorochromanteile der Antikörperkonjugate identisch sind.

10. Verwendung eines silikathaltigen Trägermaterials zur Immobilisierung von Zellen mit Analyse der Zellen, **dadurch gekennzeichnet, dass** die Zellen dadurch unspezifisch immobilisiert werden, dass das Trägermaterial Oligonukleinsäuren aufweist, die eine Abfolge von zumindest 90% Nukleotiden aufweisen, die jeweils dasselbe von A (Adenin), T (Thymin), G (Guanin), C (Cytosin), I (Inosin) oder U (Uridin) aufweisen und die eine lineare Konformation haben und die mit genau einem Ende an das Trägermaterial gebunden sind, und die auf dem Trägersubstrat immobilisierten Zellen durch Kontaktierung mit einem Nachweiskonjugat spezifisch analysiert werden, das einen für eine Komponente zumindest einer Zelle spezifischen Bindeanteil und ein Fluorochrom aufweist.

11. Verwendung nach Anspruch 10, **dadurch gekennzeichnet, dass** die Oligonukleinsäuren einsträngig sind.

12. Verwendung nach Anspruch 10, **dadurch gekennzeichnet, dass** die Oligonukleinsäuren eine doppelsträngige Konformation haben und aneinandergrenzende Abschnitte aufweisen, von denen ein erster Abschnitt aus oligo-A und/oder oligo-dA besteht, und ein zweiter Abschnitt aus oligo-T und/oder oligo-dT.

13. Verwendung nach einem der Ansprüche 10 bis 12, **dadurch gekennzeichnet, dass** die Oligonukleinsäuren linearer Konformation mittels einer Kopplungsgruppe an das Trägermaterial gebunden sind, die mit genau einem ihrer Enden verbunden ist.

14. Verwendung nach Anspruch 13, **dadurch gekennzeichnet, dass** die Kopplungsgruppe ein an das Trägersubstrat gebundenes Nukleotid ist, an welchem 2 Oligonukleinsäuren linearer Konformation gebunden sind.

15. Vorrichtung zur optischen Analyse von auf einem silikathaltigen Trägersubstrat immobilisierten Zellen, wobei die Vorrichtung einen Kanal mit einer Einlass- und einer Auslassöffnung für flüssige Zusammensetzungen aufweist, der vom Trägersubstrat, einem von diesem beabstandeten Deckel und zwischen Trägersubstrat und Deckel angeordneten Abstandshaltern gebildet wird, **dadurch gekennzeichnet, dass** ein Abschnitt des Trägersubstrats mit Oligonukleinsäuren beschichtet ist, die eine Abfolge von zumindest 90% Nukleotiden aufweisen, die jeweils dasselbe von G (Guanin), C (Cytosin), I (Inosin) oder U (Uridin) aufweisen und die im wässrigen Medium eine lineare Konformation aufweisen und mit genau einem ihrer Enden an das Trägersubstrat gebunden sind, wobei die Vorrichtung eine Aufzeichnungseinheit zur Aufzeichnung mikroskopischer Bilder des Trägersubstrats aufweist, einen elektronischen Speicher zur Speicherung der aufgezeichneten mikroskopischen Bilder, eine Rechnereinheit, die zur positionsgenauen Überlagerung von zumindest zwei aufgezeichneten mikroskopischen Bildern eingerichtet ist, und eine Anzeigeeinheit, die zur Anzeige der positionsgenau überlagerten mikroskopischen Bilder eingerichtet ist.

16. Vorrichtung nach Anspruch 15, **dadurch gekennzeichnet, dass** die Oligonukleinsäuren auf dem Abschnitt des Trägersubstrats in zumindest 5.000 Molekülen pro µm² Trägersubstrat vorliegen.

## Claims

1. Process for analysis of eucaryotic cells comprising the steps of contacting cells or tissue pieces with a section of the carrier substrate that is arranged in a canal having an inlet opening and an outlet opening,
contacting the cells arranged on the section of the carrier substrate with a first detection conjugate having a first antigen specificity and a fluorochrome portion, irradiating light having an excitation wavelength for the fluorochrome portion, detecting of the irradiation emitted by the fluorochrome portion,
storing the picture of the detected irradiation,
**characterized in that**
the section of the carrier substrate is coated with oligonucleic acids which have a sequence of at least 90% nucleotides which are the same each of A (adenine), T (thymine), G (guanine), C (cytosine), I (inosine) or U (uridine) and which in an aqueous medium have a linear confirmation and are bound to the carrier substrate with exactly one of their ends.

2. Process according to claim 1, **characterized in that** in the section of the carrier substrate the oligonucleic acids are present to at least 5,000 molecules per µm².

3. Process according to claim 1 or 2, **characterized in that** the oligonucleic acids are single-stranded.

4. Process according to claim 1 or 2, **characterized in that** the oligonucleic acids have a double-stranded conformation and have sections adjacent one another, of which a first section consists of oligo-A and/or oligo-dA and a second section consists of oligo-T and/or oligo-dT.

5. Process according to one of the preceding claims, **characterized in that** the oligonucleic acids are bound to the carrier substrate by coupling groups.

6. Process according to one of the preceding claims, **characterized in that** the carrier substrate in addition to the coating with oligonucleic acids has a frame having form fit to a tissue sample, the frame extending to a lid that is spaced apart from the carrier substrate, wherein a tissue piece is arranged in the frame.

7. Process according to one of the preceding claims, **characterized in that** subsequent to the detection of the irradiation emitted by the fluorochrome portion, the inactivation of the optical activity of the fluorochrome portion occurs and the contacting of the cells arranged on the section of the carrier substrate with a further detection conjugate having a further antigen specificity and a fluorochrome portion, irradiating light having an excitation wavelength for the fluorochrome portion, detecting of the irradiation emitted by the fluorochrome portion,
storing of the picture of the respectively detected irradiation and
inactivating the fluorochrome portion of the further detection conjugate occurs repeatly,
wherein pictures stored for each one contacting by a detection conjugate are represented virtually superimposed on one another and positionally accurate.

8. Process according to claim 7, **characterized in that** inactivating of the fluorochrome portion occurs by irradiation.

9. Process according to one of claims 7 to 8, **characterized in that** the fluorochrome portions of the antibody conjugates are identical.

10. Use of a silicate-containing carrier material for immobilisation of cells with analysis of the cells, **characterized in that** the cells are unspecifically immobilized by the carrier material having oligonucleic acids which have a sequence of the least 90% nucleotides which are each the same of A (adenine), T (thymine), G (guanine), C (cytosine), I (inosine) or U (uridine) and which have a linear conformation and which are bound to the carrier material with exactly one end, and the cells immobilized on the carrier substrate are specifically analysed by contacting with a detection conjugate which has a binding portion which is specific for a component of at least one cell and which has a fluorochrome.

11. Use according to claim 10, **characterized in that** the oligonucleic acids are single-stranded.

12. Use according to claim 10, **characterized in that** the oligonucleic acids have a double-stranded confirmation and have sections adjacent one another, of which a first section consists of oligo-A and/or of oligo-dA and a second section consists of oligo-T and/or oligo-dT.

13. Use according to one of claims 10 to 12, **characterized in that** the oligonucleic acids having linear conformation are bound to the carrier material by means of a coupling group which is bound with exactly one of their ends.

14. Use according to claim 13, **characterized in that** the coupling group is a nucleotide bound to the carrier substrate, on which nucleotide two oligonucleic acids of linear conformation are bound.

15. Device for optical analysis of cells immobilized on a silicate-containing carrier substrate, wherein the device has a canal having an inlet opening and an outlet opening for liquid compositions, the canal being formed of the carrier substrate, a lid spaced therefrom and spacers arranged between the carrier substrate and the lid, **characterized in that** a section of the carrier substrate is coated with oligonucleic acids having a sequence of the least 90% nucleotides, which are each the same of A (adenine), T (thymine), G (guanine), C (cytosine), I (inosine) or U (uridine), and which in an aqueous medium have a linear confirmation and are bound with exactly one of their ends to the carrier substrate, wherein the device has a recording unit for recording microscopic pictures of the carrier substrate, an electronic storage for storing the recorded microscopic pictures, a calculating unit which is disposed for a positionally accurate superimposition of at least two recorded microscopic pictures, and a display unit, disposed for display of the positionally accurate superimposed microscopic pictures.

16. Device according to claim 15, **characterized in that** the oligonucleic acids are present on the section of the carrier substrate to at least 5,000 molecules per µm² of the carrier substrate.

## Revendications

1. Procédé d'analyse de cellules eucaryotes comprenant les phases de mise en contact de cellules ou de fragments de tissu avec une partie d'un substrat de support, partie disposée dans un conduit présentent une ouverture d'entrée et une ouverture de sortie,
la mise en contact des cellules disposées sur la partie du substrat de support avec un premier conjugué de détection, conjugué présentant une première spécificité antigénique et une portion de fluorochrome,
le rayonnement de lumière d'une longueur d'ondes d'excitation pour la portion de fluorochrome,
la détection du rayonnement émis par la portion de fluorochrome,
le stockage de l'image du rayonnement détecté,
**caractérisé en ce que**
la partie du substrat de support est recouverte d'acides oligonucléiques, présentant une séquence d'au moins 90% de nucléotides, comportant respectivement le même élément choisi parmi A (adénine), T (thymine), G (guanine), C (cytosine), I (inosine) ou U (uridine) et présentant une conformation linéaire dans le milieu aqueux et qui sont reliés au substrat de support par exactement l'une de leurs extrémités.

2. Procédé selon la revendication 1, **caractérisé en ce que** les acides oligonucléiques sont présents à raison d'au moins 5000 molécules par µm² dans la partie du substrat de support.

3. Procédé selon la revendication 1 ou 2, **caractérisé en ce que** les acides oligonucléiques sont à simple brin.

4. Procédé selon la revendication 1 ou 2, **caractérisé en ce que** les acides oligonucléiques ont une configuration à double brin et présentent des portions limitrophes dont une première portion se compose d'oligo A et/ou d'oligo dA, et une seconde portion d'oligo-T et/ou d'oligo-dT.

5. Procédé selon l'une des revendications précédentes, **caractérisé en ce que** les acides oligonucléiques sont liés au substrat de support par le biais de groupes de liaison.

6. Procédé selon l'une des revendications précédentes, **caractérisé en ce que** le substrat de support, outre l'enduction avec des acides oligonucléiques, présente un cadre lié à un échantillon de tissu par concordance des formes, cadre s'étendant jusqu'à un couvercle espacé du substrat de support, où un fragment de tissu est disposé dans le cadre.

7. Procédé selon l'une des revendications précédentes, **caractérisé en ce que** suite à la détection du rayonnement émis par la portion de fluorochrome, l'activité optique de la portion de fluorochrome est neutralisée et on observe de nouveau la mise en contact des cellules disposées sur la portion du substrat de support avec un autre conjugué de détection, qui comporte une autre spécificité antigénique et une portion de fluorochrome, le rayonnement de lumière d'une longueur d'ondes d'excitation pour la portion de fluorochrome,
la détection du rayonnement émis par la portion de fluorochrome,
le stockage de l'image du rayonnement respectivement détecté et la neutralisation de la portion de fluorochrome du conjugué de détection supplémentaire, où pour chaque mise en contact avec un conjugué de détection, les images stockées sont représentées virtuellement en superposition avec un positionnement précis.

8. Procédé selon la revendication 7, **caractérisé en ce que** la neutralisation de la portion de fluorochrome s'effectue par rayonnement.

9. Procédé selon l'une des revendications 7 à 8, **caractérisé en ce que** les portions de fluorochrome des conjugués d'anticorps sont identiques.

10. Utilisation d'un matériau support contenant du silicate pour immobilisation des cellules avec analyse des cellules, **caractérisée en ce que** les cellules sont immobilisées de manière non spécifique de telle sorte que le matériau support contient des acides oligonucléiques, présentant une séquence d'au moins 90% de nucléotides, comportant respectivement le même élément choisi parmi A (adénine), T (thymine), G (guanine), C (cytosine, I (inosine) ou U (uridine) et ayant une conformation linéaire et qui sont liés au matériau support par exactement une extrémité, et les cellules immobilisées sur le substrat de support sont analysées par mise en contact avec un conjugué de détection de manière spécifique, qui présente un fluorochrome et une portion de liaison spécifique pour un composant d'au moins une cellule.

11. Utilisation selon la revendication 10, **caractérisée en ce que** les acides oligonucléiques sont à simple brin.

12. Utilisation selon la revendication 10, **caractérisée en ce que** les acides oligonucléiques ont une configuration à double brin et présentent des portions limitrophes dont une première portion se compose d'oligo A et/ou d'oligo dA, et une seconde portion d'oligo-T et/ou d'oligo-dT.

13. Utilisation selon l'une des revendications 10 à 12, **caractérisée en ce que** les acides oligonucléiques de conformation linéaire sont liés au matériau support à l'aide d'un groupe de liaison, groupe lié exactement en l'une de leurs extrémités.

14. Utilisation selon la revendication 13, **caractérisée en ce que** le groupe de liaison est un nucléotide lié au substrat de support, nucléotide auquel sont liés 2 acides oligonucléiques de conformation linéaire.

15. Dispositif d'analyse optique de cellules immobilisées sur un substrat de support contenant du silicate, où le dispositif contient un conduit avec une ouverture d'entrée et une ouverture de sortie pour compositions liquides, conduit constitué du substrat de support, d'un couvercle espacé de celui-ci et d'entretoises aménagées entre le substrat de support et le couvercle, **caractérisé en ce qu'**une portion du substrat de support est revêtue d'acides oligonucléiques, présentant une séquence d'au moins 90% de nucléotides, comprenant le même élément parmi G (guanine), C (cytosine), I (inosine) ou U (uridine) et présentant une conformation linéaire dans le milieu aqueux et qui sont reliés au substrat de support par exactement l'une de leurs extrémités, où le dispositif présente une unité d'enregistrement permettant d'enregistrer des images microscopiques du substrat de support, une mémoire électronique permettant de stocker les images microscopiques enregistrées, une unité de calcul, qui est configurée pour la superposition avec positionnement précis d'au moins deux images microscopiques enregistrées et une unité d'affichage, qui est configurée pour afficher des images microscopiques superposées de manière positionnement précise.

16. Dispositif selon la revendication 15, **caractérisé en ce que** les acides oligonucléiques sont présents sur la portion du substrat de support dans au moins 5000 molécules par µm² de substrat de support.
